# EUROPEAN PATENT APPLICATION

(11) **EP 1 394 250 A1**
(43) Date of publication of application: **03.03.2004**
(21) Application number: 02724764.2
(22) Date of filing: 10.05.2002
(51) Int. Cl.: C12N 15/09, C07K 14/00, C07K 19/00, C12Q 1/68, G01N 33/53

(54) **METHOD OF FORMING STABLE COMPLEX OF TRANSCRIPTION PRODUCT OF DNA ENCODING ARBITRARY PEPTIDE WITH TRANSLATION PRODUCT, NUCLEIC ACID CONSTRUCT TO BE USED IN THE METHOD, COMPLEX FORMED BY THE METHOD ; AND SCREENING OF FUNCTIONAL PROTEIN AND MRNA OR DNA ENCODING THE PROTEIN USING THE METHOD**

(30) Priority: 11.05.2001 JP 2001141594; 07.03.2002 JP 2002062751
(71) Applicant: Taira, Kazunari, Tsukuba-shi, Ibaraki 305-0046 (JP); Fujita, Satoshi, Tsukuba-shi, Ibaraki 305-0061 (JP); Yoshizaki, Shinya, Tsukuba-shi, Ibaraki 305-0061 (JP); Warashina, Masaki, Fujieda-shi, Shizuoka 426-0062 (JP)
(72) Inventor: Taira, Kazunari, Tsukuba-shi, Ibaraki 305-0046 (JP); Fujita, Satoshi, Tsukuba-shi, Ibaraki 305-0061 (JP); Yoshizaki, Shinya, Tsukuba-shi, Ibaraki 305-0061 (JP); Warashina, Masaki, Fujieda-shi, Shizuoka 426-0062 (JP)
(74) Representative: Woods, Geoffrey Corlett
(86) International application number: PCT/JP2002/004582
(87) International publication number: WO 2002/101036

(57) **Abstract**

A stable linkage between a genotype and a phenotype in a cell-free system was successfully achieved by using interaction between a RNA-binding protein and RNA, between a DNA-binding protein and DNA, or by using a protein that inactivates a ribosome. Furthermore, it was found that functional proteins could be selected by using these stable linkages.

SEQUENCE LISTING

<110> TAIRA Kazunari

FUJITA Satoshi

YOSHIZAKI Shinya

WARASHINA Masaki

<120> METHODS FOR FORMING A STABLE COMPLEX BETWEEN THE TRANSCRIPT AND
TRANSLATION PRODUCT OF A DNA ENCODING AN ARBITRARY POLYPEPTIDE, NUCLEIC ACID
CONSTRUCTS TO BE USED IN THESE METHODS, COMPLEXES FORMED BY THE METHODS, USE
OF THESE METHODS TO SCREEN FOR FUNCTIONAL PROTEINS, AND mRNAs AND DNAs
ENCODING THESE PROTEINS

<130> SEN-A0117Y1P

<140>
<141>

<150> JP 2001-141594
<151> 2001-5-11

<150> JP 2002-62751
<151> 2002-3-7

<160> 24

<210> 1

<211> 8155

<212> DNA

<213> Artificial Sequence

<220>

<223> Description of Artificial Sequence: a recombinant sequence

<400> 1

tggcgaatgg gacgcgccct gtagcggcgc attaagcgcg gcgggtgtgg tggttacgcg 60

cagcgtgacc gctacacttg ccagcgccct agcgcccgct cctttcgctt tcttcccttc 120

ctttctcgcc acgttcgccg gctttccccg tcaagctcta aatcgggggc tccctttagg 180

gttccgattt agtgctttac ggcacctcga ccccaaaaaa cttgattagg gtgatggttc 240

acgtagtggg ccatcgccct gatagacggt ttttcgccct ttgacgttgg agtccacgtt 300

ctttaatagt ggactcttgt tccaaactgg aacaacactc aaccctatct cggtctattc 360

ttttgattta taagggattt tgccgatttc ggcctattgg ttaaaaaatg agctgattta 420

acaaaaattt aacgcgaatt ttaacaaaat attaacgttt acaatttcag gtggcacttt 480

tcggggaaat gtgcgcggaa cccctatttg tttatttttc taaatacatt caaatatgta 540

tccgctcatg aattaattct tagaaaaact catcgagcat caaatgaaac tgcaatttat 600

tcatatcagg attatcaata ccatattttt gaaaaagccg tttctgtaat gaaggagaaa 660

actcaccgag gcagttccat aggatggcaa gatcctggta tcggtctgcg attccgactc 720

gtccaacatc aatacaacct attaatttcc cctcgtcaaa aataaggtta tcaagtgaga 780

aatcaccatg agtgacgact gaatccggtg agaatggcaa aagtttatgc atttctttcc 840

agacttgttc aacaggccag ccattacgct cgtcatcaaa atcactcgca tcaaccaaac 900

cgttattcat tcgtgattgc gcctgagcga gacgaaatac gcgatcgctg ttaaaaggac 960

aattacaaac aggaatcgaa tgcaaccggc gcaggaacac tgccagcgca tcaacaatat 1020

tttcacctga atcaggatat tcttctaata cctggaatgc tgttttcccg gggatcgcag 1080

tggtgagtaa ccatgcatca tcaggagtac ggataaaatg cttgatggtc ggaagaggca 1140
taaattccgt cagccagttt agtctgacca tctcatctgt aacatcattg gcaacgctac 1200
ctttgccatg tttcagaaac aactctggcg catcgggctt cccatacaat cgatagattg 1260
tcgcacctga ttgcccgaca ttatcgcgag cccatttata cccatataaa tcagcatcca 1320
tgttggaatt taatcgcggc ctagagcaag acgtttcccg ttgaatatgg ctcataacac 1380
cccttgtatt actgtttatg taagcagaca gttttattgt tcatgaccaa aatcccttaa 1440
cgtgagtttt cgttccactg agcgtcagac cccgtagaaa agatcaaagg atcttcttga 1500
gatccttttt ttctgcgcgt aatctgctgc ttgcaaacaa aaaaaccacc gctaccagcg 1560
gtggtttgtt tgccggatca agagctacca actctttttc cgaaggtaac tggcttcagc 1620
agagcgcaga taccaaatac tgtccttcta gtgtagccgt agttaggcca ccacttcaag 1680
aactctgtag caccgcctac atacctcgct ctgctaatcc tgttaccagt ggctgctgcc 1740
agtggcgata agtcgtgtct taccgggttg gactcaagac gatagttacc ggataaggcg 1800
cagcggtcgg gctgaacggg gggttcgtgc acacagccca gcttggagcg aacgacctac 1860
accgaactga gatacctaca gcgtgagcta tgagaaagcg ccacgcttcc cgaagggaga 1920
aaggcggaca ggtatccggt aagcggcagg gtcggaacag gagagcgcac gagggagctt 1980
ccagggggaa acgcctggta tctttatagt cctgtcgggt ttcgccacct ctgacttgag 2040
cgtcgatttt tgtgatgctc gtcagggggg cggagcctat ggaaaaacgc cagcaacgcg 2100
gcctttttac ggttcctggc cttttgctgg ccttttgctc acatgttctt tcctgcgtta 2160
tcccctgatt ctgtggataa ccgtattacc gcctttgagt gagctgatac cgctcgccgc 2220
agccgaacga ccgagcgcag cgagtcagtg agcgaggaag cggaagagcg cctgatgcgg 2280
tattttctcc ttacgcatct gtgcggtatt tcacaccgca tatatggtgc actctcagta 2340
caatctgctc tgatgccgca tagttaagcc agtatacact ccgctatcgc tacgtgactg 2400
ggtcatggct gcgccccgac acccgccaac acccgctgac gcgccctgac gggcttgtct 2460
gctcccggca tccgcttaca gacaagctgt gaccgtctcc gggagctgca tgtgtcagag 2520
gttttcaccg tcatcaccga aacgcgcgag gcagctgcgg taaagctcat cagcgtggtc 2580
gtgaagcgat tcacagatgt ctgcctgttc atccgcgtcc agctcgttga gtttctccag 2640

aagcgttaat gtctggcttc tgataaagcg ggccatgtta agggcggttt tttcctgttt 2700
ggtcactgat gcctccgtgt aagggggatt tctgttcatg ggggtaatga taccgatgaa 2760
acgagagagg atgctcacga tacgggttac tgatgatgaa catgcccggt tactggaacg 2820
ttgtgagggt aaacaactgg cggtatggat gcggcgggac cagagaaaaa tcactcaggg 2880
tcaatgccag cgcttcgtta atacagatgt aggtgttcca cagggtagcc agcagcatcc 2940
tgcgatgcag atccggaaca taatggtgca gggcgctgac ttccgcgttt ccagacttta 3000
cgaaacacgg aaaccgaaga ccattcatgt tgttgctcag gtcgcagacg ttttgcagca 3060
gcagtcgctt cacgttcgct cgcgtatcgg tgattcattc tgctaaccag taaggcaacc 3120
ccgccagcct agccgggtcc tcaacgacag gagcacgatc atgcgcaccc gtggggccgc 3180
catgccggcg ataatggcct gcttctcgcc gaaacgtttg gtggcgggac cagtgacgaa 3240
ggcttgagcg agggcgtgca agattccgaa taccgcaagc gacaggccga tcatcgtcgc 3300
gctccagcga aagcggtcct cgccgaaaat gacccagagc gctgccggca cctgtcctac 3360
gagttgcatg ataaagaaga cagtcataag tgcggcgacg atagtcatgc cccgcgccca 3420
ccggaaggag ctgactgggt tgaaggctct caagggcatc ggtcgagatc ccggtgccta 3480
atgagtgagc taacttacat taattgcgtt gcgctcactg cccgctttcc agtcgggaaa 3540
cctgtcgtgc cagctgcatt aatgaatcgg ccaacgcgcg gggagaggcg gtttgcgtat 3600
tgggcgccag ggtggttttt cttttcacca gtgagacggg caacagctga ttgcccttca 3660
ccgcctggcc ctgagagagt tgcagcaagc ggtccacgct ggtttgcccc agcaggcgaa 3720
aatcctgttt gatggtggtt aacggcggga tataacatga gctgtcttcg gtatcgtcgt 3780
atcccactac cgagatgtcc gcaccaacgc gcagcccgga ctcggtaatg gcgcgcattg 3840
cgcccagcgc catctgatcg ttggcaacca gcatcgcagt gggaacgatg ccctcattca 3900
gcatttgcat ggtttgttga aaaccggaca tggcactcca gtcgccttcc cgttccgcta 3960
tcggctgaat ttgattgcga gtgagatatt tatgccagcc agccagacgc agacgcgccg 4020
agacagaact taatgggccc gctaacagcg cgatttgctg gtgacccaat gcgaccagat 4080
gctccacgcc cagtcgcgta ccgtcttcat gggagaaaat aatactgttg atgggtgtct 4140
ggtcagagac atcaagaaat aacgccggaa cattagtgca ggcagcttcc acagcaatgg 4200

catcctggtc atccagcgga tagttaatga tcagcccact gacgcgttgc gcgagaagat 4260

tgtgcaccgc cgctttacag gcttcgacgc cgcttcgttc taccatcgac accaccacgc 4320

tggcacccag ttgatcggcg cgagatttaa tcgccgcgac aatttgcgac ggcgcgtgca 4380

gggccagact ggaggtggca acgccaatca gcaacgactg tttgcccgcc agttgttgtg 4440

ccacgcggtt gggaatgtaa ttcagctccg ccatcgccgc ttccactttt tcccgcgttt 4500

tcgcagaaac gtggctggcc tggttcacca cgcgggaaac ggtctgataa gagacaccgg 4560

catactctgc gacatcgtat aacgttactg gtttcacatt caccaccctg aattgactct 4620

cttccgggcg ctatcatgcc ataccgcgaa aggttttgcg ccattcgatg gtgtccggga 4680

tctcgacgct ctcccttatg cgactcctgc attaggaagc agcccagtag taggttgagg 4740

ccgttgagca ccgccgccgc aaggaatggt gcatgcaagg agatggcgcc caacagtccc 4800

ccggccacgg ggcctgccac catacccacg ccgaaacaag cgctcatgag cccgaagtgg 4860

cgagcccgat cttccccatc ggtgatgtcg gcgatatagg cgccagcaac cgcacctgtg 4920

gcgccggtga tgccggccac gatgcgtccg gcgtagagga tcgagatcga tctcgatccc 4980

gcgaaattaa tacgactcac tataggggaa ttgtgagcgg ataacaattc ccctctagaa 5040

ataattttgt ttaactttaa gaaggagatat acatatggc gagcatgact ggtggacagc 5100

aaatgggtac cgaattccat atggacccgtc caaggactc caaagctcag gtttctgcag 5160

ccgaagctgg tatcactggc acctggtataa ccaactggg gtcgactttc attgtgaccg 5220

ctggtgcgga cggagctctg actggcaccta cgaatctgc ggttggtaac gcagaatccc 5280

gctacgtact gactggccgt tatgactctgc acctgccac cgatggctct ggtaccgctc 5340

tgggctggac tgtggcttgg aaaaacaacta tcgtaatgc gcacagcgcc actacgtggt 5400

ctggccaata cgttggcggt gctgaggctcg tatcaacac tcagtggctg ttaacatccg 5460

gcactaccga agcgaatgca tggaaatcgac actagtagg tcatgacacc tttaccaaag 5520

ttaagccttc tgctgcgagc attgatgctgc caagaaagc aggcgtaaac aacggtaacc 5580

ctcttgacgc tgttcagcaa gctagccctg tcaatgctgg cggcggctct ggtggtggtt 5640

ctggtggcgg ctctgagggt ggcggctctg agggtggcgg ctctgacggt ggcggttctg 5700

agggtggcgg ctctgagggt ggcggttccg gtggcggctc cggttccggt gattttgatt 5760

atccatggat gatatttccc aaacaatacc caattataaa ctttaccaca gcgggtgcca 5820
ctgtgcaaag ctacacaaac tttatcagag ctgttcgcgg tcgtttaaca actggagctg 5880
atgtgagaca tgaaatacca gtgttgccaa acagagttgg tttgcctata aaccaacggt 5940
ttattttagt tgaactctca aatcatgcag agctttctgt tacattagcg ctggatgtca 6000
ccaatgcata tgtggtaggc taccgtgctg gaaatagcgc atatttcttt catcctgaca 6060
atcaggaaga tgcagaagca atcacttatc ttttcactga tgttcaaaat cgatatacat 6120
tcgcctttgg tggtaattat gatagacttg aacaacttgc tggtaatctg agagaaaata 6180
tcgagttggg aaatggtcca ctagaggagg ctatctcagc gctttattat tacagtactg 6240
gtggcactca gcttccaact ctggctcgtt cctttataat ttgcatccaa atgatttcag 6300
aagcagcaag attccaatat attgagggag aaatgcgcac gagaattagg tacaaccgga 6360
gatctgcacc agatcctagc gtaattacac ttgagaatag ttgggggaga ctttccactg 6420
caattcaaga gtctaaccaa ggagcctttg ctagtccaat tcaactgcaa agacgtaatg 6480
gttccaaatt cagtgtgtac gatgtgagta tattaatccc tatcatagct ctcatggtgt 6540
atagatgcgc gcctccaccg agctcacagt ttgggatatc gtcgaccgga ggaggaggtg 6600
gcggcggtgg cgctgcatcg actgttgaaa gttgtttagc aaaacctcat acagaaaatt 6660
catttactaa cgtctggaaa gacgacaaaa ctttagatcg ttacgctaac tatgagggct 6720
gtctgtggaa tgctacaggc gttgtggttt gtactggtga cgaaactcag tgttacggta 6780
catgggttcc tattgggctt gctatccctg aaaatgaggg tggtggctct gagggtggcg 6840
gttctgaggg tggcggttct gagggtggcg gtactaaacc tcctgagtac ggtgatacac 6900
ctattccggg ctatacttat atcaaccctc tcgacggcac ttatccgcct ggtactgagc 6960
aaaaccccgc taatcctaat ccttctcttg aggagtctca gcctcttaat actttcatgt 7020
ttcagaataa taggttccga aataggcagg gtgcattaac tgtttatacg ggcactgtta 7080
ctcaaggcac tgaccccgtt aaaacttatt accagtacac tcctgtatca tcaaaagcca 7140
tgtatgacgc ttactggaac ggtaaattca gagactgcgc tttccattct ggctttaatg 7200
aggatccatt cgtttgtgaa tatcaaggcc aatcgtctga cctgcctcaa cctcctgtca 7260
atgctggcgg cggctctggt ggtggttctg gtggcggctc tgagggtggc ggctctgagg 7320

gtggcggttc tgagggtggc ggctctgagg gtggcggttc cggtggcggc tccggttccg 7380

gtgattttga ttatgaaaaa atggcaaacg ctaataaggg ggctatgacc gaaaatgccg 7440

atgaaaacgc gctacagtct gacgctaaag gcaaacttga ttctgtcgct actgattacg 7500

gtgctgctat cgatggtttc attggtgacg tttccggcct tgctaatggt aatggtgcta 7560

ctggtgattt tgctggctct aattcccaaa tggctcaagt cggtgacggt gataattcac 7620

ctttaatgaa taatttccgt caatatttac cttctttgcc tcagtcggtt gaatgtcgcc 7680

cttatgtctt tggcgctggt aaaccatatg aattttctat tgattgtgac aaaataaact 7740

tattccgtgg tgtctttgcg tttcttttat atgttgccac ctttatgtat gtattttcga 7800

cgtttgctaa catactgcgt aataaggagt ctctgcaggt taagaattcg gcttaaaaaa 7860

cgaagcttga tcccgtttgc cggtcgatcg cttcgaaaaa acgaagcttg atcccgtttg 7920

ccggtcgatc gcttcgaaaa aacgaagctt gatcccgttt gccggtcgat cgcttcgaaa 7980

aaaagccgaa ttctcgagca ccaccaccac caccactgag atccggctgc taacaaagcc 8040

cgaaaggaag ctgagttggc tgctgccacc gctgagcaat aactagcata accccttggg 8100

gcctctaaac gggtcttgag gggttttttg ctgaaaggag gaactatatc cggat 8155

<210> 2

<211> 7306

<212> DNA

<213> Artificial Sequence

<220>

<223> Description of Artificial Sequence: a recombinant sequence

<400> 2

tggcgaatgg gacgcgccct gtagcggcgc attaagcgcg gcgggtgtgg tggttacgcg 60

cagcgtgacc gctacacttg ccagcgccct agcgcccgct cctttcgctt tcttcccttc 120
ctttctcgcc acgttcgccg gctttccccg tcaagctcta aatcgggggc tccctttagg 180
gttccgattt agtgctttac ggcacctcga ccccaaaaaa cttgattagg gtgatggttc 240
acgtagtggg ccatcgccct gatagacggt ttttcgccct ttgacgttgg agtccacgtt 300
ctttaatagt ggactcttgt tccaaactgg aacaacactc aaccctatct cggtctattc 360
ttttgattta taagggattt tgccgatttc ggcctattgg ttaaaaaatg agctgattta 420
acaaaaattt aacgcgaatt ttaacaaaat attaacgttt acaatttcag gtggcacttt 480
tcggggaaat gtgcgcggaa cccctatttg tttatttttc taaatacatt caaatatgta 540
tccgctcatg aattaattct tagaaaaact catcgagcat caaatgaaac tgcaatttat 600
tcatatcagg attatcaata ccatattttt gaaaaagccg tttctgtaat gaaggagaaa 660
actcaccgag gcagttccat aggatggcaa gatcctggta tcggtctgcg attccgactc 720
gtccaacatc aatacaacct attaatttcc cctcgtcaaa aataaggtta tcaagtgaga 780
aatcaccatg agtgacgact gaatccggtg agaatggcaa aagtttatgc atttctttcc 840
agacttgttc aacaggccag ccattacgct cgtcatcaaa atcactcgca tcaaccaaac 900
cgttattcat tcgtgattgc gcctgagcga gacgaaatac gcgatcgctg ttaaaaggac 960
aattacaaac aggaatcgaa tgcaaccggc gcaggaacac tgccagcgca tcaacaatat 1020
tttcacctga atcaggatat tcttctaata cctggaatgc tgttttcccg gggatcgcag 1080
tggtgagtaa ccatgcatca tcaggagtac ggataaaatg cttgatggtc ggaagaggca 1140
taaattccgt cagccagttt agtctgacca tctcatctgt aacatcattg gcaacgctac 1200
ctttgccatg tttcagaaac aactctggcg catcgggctt cccatacaat cgatagattg 1260
tcgcacctga ttgcccgaca ttatcgcgag cccatttata cccatataaa tcagcatcca 1320
tgttggaatt taatcgcggc ctagagcaag acgtttcccg ttgaatatgg ctcataacac 1380
cccttgtatt actgtttatg taagcagaca gttttattgt tcatgaccaa aatcccttaa 1440
cgtgagtttt cgttccactg agcgtcagac cccgtagaaa agatcaaagg atcttcttga 1500
gatccttttt ttctgcgcgt aatctgctgc ttgcaaacaa aaaaaccacc gctaccagcg 1560
gtggtttgtt tgccggatca agagctacca actctttttc cgaaggtaac tggcttcagc 1620

agagcgcaga taccaaatac tgtccttcta gtgtagccgt agttaggcca ccacttcaag 1680

aactctgtag caccgcctac atacctcgct ctgctaatcc tgttaccagt ggctgctgcc 1740

agtggcgata agtcgtgtct taccgggttg gactcaagac gatagttacc ggataaggcg 1800

cagcggtcgg gctgaacggg gggttcgtgc acacagccca gcttggagcg aacgacctac 1860

accgaactga gatacctaca gcgtgagcta tgagaaagcg ccacgcttcc cgaagggaga 1920

aaggcggaca ggtatccggt aagcggcagg gtcggaacag gagagcgcac gagggagctt 1980

ccagggggaa acgcctggta tctttatagt cctgtcgggt ttcgccacct ctgacttgag 2040

cgtcgatttt tgtgatgctc gtcagggggg cggagcctat ggaaaaacgc cagcaacgcg 2100

gcctttttac ggttcctggc cttttgctgg ccttttgctc acatgttctt tcctgcgtta 2160

tcccctgatt ctgtggataa ccgtattacc gcctttgagt gagctgatac cgctcgccgc 2220

agccgaacga ccgagcgcag cgagtcagtg agcgaggaag cggaagagcg cctgatgcgg 2280

tattttctcc ttacgcatct gtgcggtatt tcacaccgca tatatggtgc actctcagta 2340

caatctgctc tgatgccgca tagttaagcc agtatacact ccgctatcgc tacgtgactg 2400

ggtcatggct gcgccccgac acccgccaac acccgctgac gcgccctgac gggcttgtct 2460

gctcccggca tccgcttaca gacaagctgt gaccgtctcc gggagctgca tgtgtcagag 2520

gttttcaccg tcatcaccga aacgcgcgag gcagctgcgg taaagctcat cagcgtggtc 2580

gtgaagcgat tcacagatgt ctgcctgttc atccgcgtcc agctcgttga gtttctccag 2640

aagcgttaat gtctggcttc tgataaagcg ggccatgtta agggcggttt tttcctgttt 2700

ggtcactgat gcctccgtgt aagggggatt tctgttcatg ggggtaatga taccgatgaa 2760

acgagagagg atgctcacga tacgggttac tgatgatgaa catgcccggt tactggaacg 2820

ttgtgagggt aaacaactgg cggtatggat gcggcgggac cagagaaaaa tcactcaggg 2880

tcaatgccag cgcttcgtta atacagatgt aggtgttcca cagggtagcc agcagcatcc 2940

tgcgatgcag atccggaaca taatggtgca gggcgctgac ttccgcgttt ccagacttta 3000

cgaaacacgg aaaccgaaga ccattcatgt tgttgctcag gtcgcagacg ttttgcagca 3060

gcagtcgctt cacgttcgct cgcgtatcgg tgattcattc tgctaaccag taaggcaacc 3120

ccgccagcct agccgggtcc tcaacgacag gagcacgatc atgcgcaccc gtggggccgc 3180

catgccggcg ataatggcct gcttctcgcc gaaacgtttg gtggcgggac cagtgacgaa 3240
ggcttgagcg agggcgtgca agattccgaa taccgcaagc gacaggccga tcatcgtcgc 3300
gctccagcga aagcggtcct cgccgaaaat gacccagagc gctgccggca cctgtcctac 3360
gagttgcatg ataaagaaga cagtcataag tgcggcgacg atagtcatgc cccgcgccca 3420
ccggaaggag ctgactgggt tgaaggctct caagggcatc ggtcgagatc ccggtgccta 3480
atgagtgagc taacttacat taattgcgtt gcgctcactg cccgctttcc agtcgggaaa 3540
cctgtcgtgc cagctgcatt aatgaatcgg ccaacgcgcg gggagaggcg gtttgcgtat 3600
tgggcgccag ggtggttttt cttttcacca gtgagacggg caacagctga ttgcccttca 3660
ccgcctggcc ctgagagagt tgcagcaagc ggtccacgct ggtttgcccc agcaggcgaa 3720
aatcctgttt gatggtggtt aacggcggga tataacatga gctgtcttcg gtatcgtcgt 3780
atcccactac cgagatgtcc gcaccaacgc gcagcccgga ctcggtaatg gcgcgcattg 3840
cgcccagcgc catctgatcg ttggcaacca gcatcgcagt gggaacgatg ccctcattca 3900
gcatttgcat ggtttgttga aaaccggaca tggcactcca gtcgccttcc cgttccgcta 3960
tcggctgaat ttgattgcga gtgagatatt tatgccagcc agccagacgc agacgcgccg 4020
agacagaact taatgggccc gctaacagcg cgatttgctg gtgacccaat gcgaccagat 4080
gctccacgcc cagtcgcgta ccgtcttcat gggagaaaat aatactgttg atgggtgtct 4140
ggtcagagac atcaagaaat aacgccggaa cattagtgca ggcagcttcc acagcaatgg 4200
catcctggtc atccagcgga tagttaatga tcagcccact gacgcgttgc gcgagaagat 4260
tgtgcaccgc cgctttacag gcttcgacgc cgcttcgttc taccatcgac accaccacgc 4320
tggcacccag ttgatcggcg cgagatttaa tcgccgcgac aatttgcgac ggcgcgtgca 4380
gggccagact ggaggtggca acgccaatca gcaacgactg tttgcccgcc agttgttgtg 4440
ccacgcggtt gggaatgtaa ttcagctccg ccatcgccgc ttccactttt tcccgcgttt 4500
tcgcagaaac gtggctggcc tggttcacca cgcgggaaac ggtctgataa gagacaccgg 4560
catactctgc gacatcgtat aacgttactg gtttcacatt caccaccctg aattgactct 4620
cttccgggcg ctatcatgcc ataccgcgaa aggttttgcg ccattcgatg gtgtccggga 4680
tctcgacgct ctcccttatg cgactcctgc attaggaagc agcccagtag taggttgagg 4740

ccgttgagca ccgccgccgc aaggaatggt gcatgcaagg agatggcgcc caacagtccc 4800

ccggccacgg ggcctgccac catacccacg ccgaaacaag cgctcatgag cccgaagtgg 4860

cgagcccgat cttccccatc ggtgatgtcg gcgatatagg cgccagcaac cgcacctgtg 4920

gcgccggtga tgccggccac gatgcgtccg gcgtagagga tcgagatcga tctcgatccc 4980

gcgaaattaa tacgactcac tataggggaa ttgtgagcgg ataacaattc ccctctagaa 5040

ataattttgt ttaactttaa gaaggagata tacatatggc gagcatgact ggtggacagc 5100

aaatgggtac cgaattccat atggacccgt ccaaggactc caaagctcag gtttctgcag 5160

ccgaagctgg tatcactggc acctggtata accaactggg gtcgactttc attgtgaccg 5220

ctggtgcgga cggagctctg actggcacct acgaatctgc ggttggtaac gcagaatccc 5280

gctacgtact gactggccgt tatgactctg cacctgccac cgatggctct ggtaccgctc 5340

tgggctggac tgtggcttgg aaaaacaact atcgtaatgc gcacagcgcc actacgtggt 5400

ctggccaata cgttggcggt gctgaggctc gtatcaacac tcagtggctg ttaacatccg 5460

gcactaccga agcgaatgca tggaaatcga cactagtagg tcatgacacc tttaccaaag 5520

ttaagccttc tgctgcgagc attgatgctg ccaagaaagc aggcgtaaac aacggtaacc 5580

ctcttgacgc tgttcagcaa gctagccctg tcaatgctgg cggcggctct ggtggtggtt 5640

ctggtggcgg ctctgagggt ggcggctctg agggtggcgg ctctgacggt ggcggttctg 5700

agggtggcgg ctctgagggt ggcggttccg gtggcggctc cggttccggt gattttgatt 5760

atccatggat gatatttccc aaacaatacc caattataaa ctttaccaca gcgggtgcca 5820

ctgtgcaaag ctacacaaac tttatcagag ctgttcgcgg tcgtttaaca actggagctg 5880

atgtgagaca tgaaatacca gtgttgccaa acagagttgg tttgcctata aaccaacggt 5940

ttattttagt tgaactctca aatcatgcag agctttctgt tacattagcg ctggatgtca 6000

ccaatgcata tgtggtaggc taccgtgctg gaaatagcgc atatttcttt catcctgaca 6060

atcaggaaga tgcagaagca atcacttatc ttttcactga tgttcaaaat cgatatacat 6120

tcgcctttgg tggtaattat gatagacttg aacaacttgc tggtaatctg agagaaaata 6180

tcgagttggg aaatggtcca ctagaggagg ctatctcagc gctttattat tacagtactg 6240

gtggcactca gcttccaact ctggctcgtt cctttataat ttgcatccaa atgatttcag 6300

aagcagcaag attccaatat attgagggag aaatgcgcac gagaattagg tacaaccgga 6360

gatctgcacc agatcctagc gtaattacac ttgagaatag ttgggggaga ctttccactg 6420

caattcaaga gtctaaccaa ggagcctttg ctagtccaat tcaactgcaa agacgtaatg 6480

gttccaaatt cagtgtgtac gatgtgagta tattaatccc tatcatagct ctcatggtgt 6540

atagatgcgc gcctccaccg agctcacagt ttgggatatc gtcgaccgga ggaggaggtg 6600

gcggcggtgg cgctgcatcg gatccattcg tttgtgaata tcaaggccaa tcgtctgacc 6660

tgcctcaacc tcctgtcaat gctggcggcg gctctggtgg tggttctggt ggcggctctg 6720

agggtggcgg ctctgagggt ggcggttctg agggtggcgg ctctgagggt ggcggttccg 6780

gtggcggctc cggttccggt gattttgatt atgaaaaaat ggcaaacgct aataaggggg 6840

ctatgaccga aaatgccgat gaaaacgcgc tacagtctga cgctaaaggc aaacttgatt 6900

ctgtcgctac tgattacggt gctgctatcg atggtttcat tggtgacgtt tccggccttg 6960

ctaatggtaa tggtgctact ggtctgcagg ttaagaattc ggcttaaaaa acgaagcttg 7020

atcccgtttg ccggtcgatc gcttcgaaaa aacgaagctt gatcccgttt gccggtcgat 7080

cgcttcgaaa aaacgaagct tgatcccgtt tgccggtcga tcgcttcgaa aaaaagccga 7140

attctcgagc accaccacca ccaccactga gatccggctg ctaacaaagc ccgaaaggaa 7200

gctgagttgg ctgctgccac cgctgagcaa taactagcat aaccccttgg ggcctctaaa 7260

cgggtcttga ggggtttttt gctgaaagga ggaactatat ccggat 7306

<210> 3

<211> 38

<212> PRT

<213> Artificial Sequence

<400> 3

Arg Lys Lys Arg Arg Gln Arg Arg Arg Pro Pro Gln Gly Ser Gln Thr

1                   5                   10                  15

Asx Gln Val Ser Leu Ser Lys Gln Pro Thr Ser Gln Ser Arg Gly Asp

                20                  25                  30

Pro Thr Gly Pro Lys Glu

            35

<210> 4

<211> 41

<212> DNA

<213> Artificial Sequence

<220>

<223> Description of Artificial Sequence: an artificial synthesized DNA

<400> 4

aaaaaacgaa gcttgatccc gtttgccggt cgatcgcttc g                          41

<210> 5

<211> 40

<212> DNA

<213> Artificial Sequence

<220>

<223> Description of Artificial Sequence: an artificial synthesized DNA

<400> 5

tttttcgaa gcgatcgacc ggcaaacggg atcaagcttc                                40

<210> 6

<211> 20

<212> DNA

<213> Artificial Sequence

<220>

<223> Description of Artificial Sequence: an artificial synthesized DNA

<400> 6

acaattcccc tctagaaata                                                     20

<210> 7

<211> 20

<212> DNA

<213> Artificial Sequence

<220>

<223> Description of Artificial Sequence: an artificial synthesized DNA

<400> 7

gtggtggtgc tcgagaattc                                                     20

<210> 8

<211> 42

<212> DNA

<213> Artificial Sequence

<220>

<223> Description of Artificial Sequence: an artificial synthesized DNA

<400> 8

cctgccgccc tcgcctgggc caaacgcaac accttaaata aa 42

<210> 9

<211> 39

<212> DNA

<213> Artificial Sequence

<220>

<223> Description of Artificial Sequence: an artificial synthesized DNA

<400> 9

gcgtttggcc caggcgaggg cggcaggcag gttccacgg 39

<210> 10

<211> 36

<212> PRT

<213> Artificial Sequence

<400> 10

Cys Phe Thr Thr Lys Ala Leu Gly Ile Ser Tyr Gly Arg Lys Lys Arg

1               5                   10                  15

Arg Gln Arg Arg Arg Pro Pro Gln Gly Ser Gln Thr Asx Gln Val Ser

            20                  25                  30

Leu Ser Lys Gln

        35

<210> 11

<211> 39

<212> DNA

<213> Artificial Sequence

<220>

<223> Description of Artificial Sequence: an artificial synthesized DNA

<400> 11

gtggcgatgc atcgactgtt gaaagttgtt tagcaaaac 39

<210> 12

<211> 38

<212> DNA

<213> Artificial Sequence

<220>

<223> Description of Artificial Sequence: an artificial synthesized DNA

<400> 12

ttcttactgc agagactcct tattacgcag tatgttag 38

<210> 13

<211> 39

<212> DNA

<213> Artificial Sequence

<220>

<223> Description of Artificial Sequence: an artificial synthesized DNA

<400> 13

gtggcgatgc atcggatcca ttcgtttgtg aatatcaag 39

<210> 14

<211> 38

<212> DNA

<213> Artificial Sequence

<220>

<223> Description of Artificial Sequence: an artificial synthesized DNA

<400> 14

ttcttactgc agaccagtag caccattacc attagcaa 38

<210> 15

<211> 38

<212> DNA

<213> Artificial Sequence

<220>

<223> Description of Artificial Sequence: an artificial synthesized DNA

<400> 15

actcggagta cttgctagcc ctgtcaatgc tggcggcg 38

<210> 16

<211> 60

<212> DNA

<213> Artificial Sequence

<220>

<223> Description of Artificial Sequence: an artificial synthesized DNA

<400> 16

gctacggata tcccgcttgt aacaggagtg ctccatggat aatcaaaatc accggaaccg    60

<210> 17

<211> 35

<212> DNA

<213> Artificial Sequence

<220>

<223> Description of Artificial Sequence: an artificial synthesized DNA

<400> 17

cgcgcgccat ggatgtttcc caaacaatac ccaat                               35

<210> 18

<211> 39

<212> DN[?]

<213> Artificial Sequence

<220>

<223> Description of Artificial Sequence: an artificial synthesized DNA

<400> 18
ggggcggata tcccaaactg tgagctcggt ggaggcgcg 39

<210> 19
<211> 71
<212> DNA
<213> Artificial Sequence

<220>
<223> Description of Artificial Sequence: an artificial synthesized DNA

<400> 19
ggccggtcta gaaataattt tgtttaactt taagaaggag atatacatat gtcccctata 60
ctaggttatt g 71

<210> 20
<211> 38
<212> DNA
<213> Artificial Sequence

<220>
<223> Description of Artificial Sequence: an artificial synthesized DNA

<400> 20

cggtgggcta gccagatccg attttggagg atggtcgc 38

<210> 21

<211> 71

<212> DNA

<213> Artificial Sequence

<220>

<223> Description of Artificial Sequence: an artificial synthesized DNA

<400> 21

ggccggtcta gaaataattt tgtttaactt taagaaggag atatacatat gcaccatcat 60

catcatcatt c 71

<210> 22

<211> 36

<212> DNA

<213> Artificial Sequence

<220>

<223> Description of Artificial Sequence: an artificial synthesized DNA

<400> 22

cggtgggcta gcccgccgct ccagaatctc aaagca                                36

<210> 23

<211> 24

<212> DNA

<213> Artificial Sequence

<220>

<223> Description of Artificial Sequence: an artificial synthesized DNA

<400> 23

ctttaagaag gagatataca tatg                                             24

<210> 24

<211> 20

<212> DNA

<213> Artificial Sequence

<220>

<223> Description of Artificial Sequence: an artificial synthesized DNA

<400> 24

ctcctccggt cgacgatatc                                                  20

## Description

### Technical Field

The present invention relates to methods for forming a complex between the transcript and translation product of a DNA encoding an arbitrary polypeptide, nucleic acid constructs to be used in these methods, complexes formed by the methods, use of these methods to screen for functional proteins, and mRNAs and DNAs encoding these proteins.

### Background Art

Recent years have seen an increase in the importance of a method for selecting and identifying a functional protein from a group of random amino acid sequences. Living cells are used in many screening systems, such as the phage display method and the two hybrid method, which can be used for efficient selection of.specific.functional proteins (Fields, S. *et al*., Nature, 1989, 340, 245-246; Harada, K. *et al*., Nature, 1996, 380, 175-179; Moore, J. C. *et al*., Nature Biotech., 1996, 14, 458-467; Schatz, P. J. *et al*., Methods Enzymol., 1996, 267, 171-191; Boder, E. T. *et al*., Nature Biotech., 1997, 15, 553-557; Smith, G. P. *et al*., Chem. Rev., 1997, 97, 391-410). In such selection systems, the sequence information (genotype) encoding a selected·protein (phenotype) is obtained from a DNA that has been introduced into each cell displaying the appropriate phenotype. The connection between genotype and phenotype is an important factor in selecting a functional protein from a group of random sequences.

However, as long as such methods depend on the use of living cells, sequence library variety will be limited. For example, library variety will be limited due to low transfection efficiency, restricted size of proteins displayed, and restriction of target protein properties as cytotoxic proteins cannot be selected.

In order to overcome these problems, a number of methods comprising cell-free protein selection systems have been developed (Mattheakis, L. C. *et al*., Proc. Natl. Acad. Sci. USA, 1994, 91, 9022-9026; Mattheakis, L. C. *et al*., Methods Enzymol., 1996, 267, 195-207; Hanes, J. *et al*., Proc. Natl. Acad. Sci. USA, 1997, 94, 4937-4942; He, M. *et al*., Nucleic Acids Res., 1997, 25, 5132-5134; Nemoto, N. *et al*., FEES Lett., 1997, 414, 405-408; Roberts, R. W. *et al*., Proc. Natl. Acad. Sci. USA, 1997, 94, 12297-12302; Hanes, J. *et al*., Proc. Natl. Acad. Sci. USA, 1998, 95, 14130-14135; Tawfik, D. S. *et al*., Nature Biotech., 1998, 16, 652-656; Doi, N. *et al*., FEBS Lett., 1999, 457, 227-230; Hanes, J. *et al*., FEES Lett., 1999, 450, 105-110; Makeyev, E. V. *et al*., FEBS Lett., 1999, 444, 177-180; He, M. *et al*. J. Immunol. Methods, 1999, 231, 105-117; Schaffitzel, C. *et al*., J. Immunol. Methods, 1999, 231, 119-135; Hanes, J. *et al*., Nat. Biotechnol., 2000, 18, 1287-1292; Hanes, J. *et al*., Methods Enzymol., 2000, 328, 404-430).

Specifically, such methods include ribosome-display methods, methods using covalently linked protein-mRNA fusion, and micelle methods. However, currently available cell-free systems comprise processes that reduce variety, and are thus generally difficult to practically apply.

For example, when using a ribosome-display method, a ribosome is used to form a relatively stable complex between a translated protein and the mRNA encoding that protein. This stability results from a delay of protein release from the complex due to the lack of the termination codon. Under these conditions, termination factors cannot efficiently induce protein release from the ribosome complex. However, in this method, success of protein selection depends on the half life of the complex (in the absence'of the termination codon, delay of protein release is limited), and therefore selection must be carried out in a short time. In practice, maintaining a perfectly intact mRNA-ribosome-protein complex is not easy.

Ribosome display methods using Escherichia coli are described, for example, in the following references: Jermutus, L., *et al*., "Tailoring *in vitro* evolution for protein affinity or stability", Proc. Natl. Acad. Sci. USA., 2001 Jan 2, 98(1):75-80; Hanes, J. *et* *al*., "Picomolar affinity antibodies from a fully synthetic naive library selected and evolved by ribosome display", Nat. Biotechnol., 2000 Dec. 18 (12) :1287-92; Hanes, J. *et al*., "Selecting and evolving functional proteins in vitro by ribosome display", Methods Enzymol., 2000, 328:404-30; and Schaffitzel, C. *et al*., "Ribosome display: an in vitro method for selection and evolution of antibodies from libraries", J. Immunol. Methods., 1999 Dec 10;231(1-2):119-35. Ribosome display methods using rabbit reticulocyte system are described, for example, in the following references: He, M. *et al*., "Selection of a human anti-progesterone antibody fragment from a transgenic mouse library by ARM ribosome display.", J. Immunol. Methods., 1999 Dec 10, 231(1-2):105-17; and Bieberich, E. *et al*., "Protein-ribosome-mRNA display: affinity isolation of enzyme-ribosome-mRNA complexes and cDNA cloning in a single-tube reaction.", Anal Biochem., 2000 Dec 15, 287(2):294-8. A ribosome display method using the wheat germ system is described, for example, in the following reference: Gersuk, G. M. *et al*., "High-affinity peptide ligands to prostate-specific antigen identified by polysome selection", Biochem. Biophys. Res. Commun., 1997 Mar 17, 232(2):578-82.

In mRNA display, a translated protein is .covalently linked to its mRNA, whose 3'-end has been labeled with puromycin. This method typically requires chemical synthesis to link the mRNA and puromycin, a step which reduces the size of available libraries. Documents describing mRNA display include: Keefe, A. D. *et al*., "Functional proteins from a random-sequence library", Nature., 2001 Apr 5, 410(6829):715-8; Wilson, D. S. *et al*., "The use of mRNA display to select high-affinity protein-binding peptides", Proc. Natl. Acad. Sci. USA., 2001 Mar 27, 98(7):3750-5; Liu, R. *et al*., "Optimized synthesis of RNA-protein fusions for *in vitro* protein selection", Methods Enzymol., 2000, 318:268-93; and Cho, G. *et al*., "Constructing high complexity synthetic libraries of long ORFs using in vitro selection", J. Mol. Biol., 2000 Mar 24, 297(2):309-19.

In micelle methods, chemically modified DNA molecules are individually packaged into a colloidal micelle. Inside the micelle, the modified DNA is transcribed and translated, and then binds to the protein it encodes. However, because packaging is achieved by diluting the reaction mixture such that each micelle contains a single DNA molecule, the sequence variety of the library is reduced. Thus, cell-free systems have an advantage over systems that use living cells in that they can test more sequences. However, a considerable portion of the sequence pool remains to be sufficiently searched using the currently available cell-free systems.

### Disclosure of the Invention

The present invention was achieved under the above circumstances. An objective of the present invention is to provide methods that can select functional proteins from a group of random amino acid sequences without reducing sequence variety. Another objective of the present invention is to provide methods that enable the formation of a stable linkage between genotype and phenotype in a cell-free system, to enable selection of such functional proteins. Yet another objective of the present invention is to provide nucleic acid constructs that can be used to form a stable linkage between genotype and phenotype.

The present inventors aimed to form a stable linkage between genotype and phenotype in a cell-free system to achieve the objectives described above.

The first method comprises using the strong interaction between an RNA-binding protein and RNA to form a stable linkage between genotype and phenotype. As an example, an aptamer (referred to as the Tat aptamer), which binds HIV-1 Tat (trans activation) protein more strongly than the RNA interacting with that Tat protein, was selected: The Tat protein is known as the trans-activating response region (TAR). (Yamamoto, R. *et al*., Genes Cells, 2000, 5, 371-388). The interaction between the Tat aptamer and a Tat-derived peptide (the RE peptide (38 amino acids) or CQ peptide (36 amino acids)) is very strong, and the dissociation constant (Kd) is in the range of nanomoles or less. To produce a linkage between genotype and phenotype using this interaction, the present inventors prepared a DNA construct in which the Tat aptamer and a DNA encoding a Tat-derived peptide were ligated with the DHFR gene. They then tested whether the transcript (mRNA) and the DHFR gene translation product (protein) were able to form a stable complex.

These results revealed that interaction between the Tat aptamer and a Tat-derived peptide is useful in linking the DHFR gene genotype and phenotype, i.e. the transcript and translation product, as long as the complex does not form a network structure. The increased number of aptamers and peptide motifs in the DNA construct, and DNA construct modifications such as deletion of the termination codon in the transcript, were effective in improving complex stability.

As an alternative example, genotype and phenotype were linked using the interaction between the MS2 coat protein dimer and the RNA sequence of a specific binding motif corresponding to that protein. This resulted in the formation of a stable complex between the transcript and translation product for the MS2 coat protein gene.

Furthermore, the present inventors conceived that interaction between a DNA-binding protein and DNA could be used to achieve stable linkage between genotype and phenotype, instead of the above-described interaction between an RNA-binding protein and RNA. Specifically, it was thought that if a nucleic acid construct could be constructed such that it comprised a complex of i) an mRNA encoding a chimeric peptide formed from a DNA-binding protein and an arbitrary peptide; and ii) a DNA comprising a binding region for that DNA-binding protein, and the mRNA in the nucleic acid construct were to be translated, the translation product might bind to the DNA in the nucleic acid construct, thereby forming a stable complex between the translation product and the nucleic acid construct.

The second method comprises using ribosome inactivation to help form a stable linkage between genotype and phenotype.. The ricin A chain was chosen as one example for ribosome inactivation. Ricin is a castor seed-derived cytotoxic protein which terminates protein synthesis by inactivating ribosomes. The ricin A chain subunit highly specifically hydrolyzes the 28S rRNA N-glucoside linkage at its alpha-salicin site, thus inactivating ribosomes. In rat 28S rRNA, the adenosine at nucleotide 4,324 is hydrolyzed. Ribosomes inactivated by ricin stop on the mRNA chain. To link genotype with phenotype using this ricin A chain-ribosome interaction, the present inventors prepared DNA constructs in which DNA encoding the ricin A chain was ligated with the DHFR gene, the GST gene, or the streptavidin gene, and then examined whether a stable complex could be formed from the transcript (mRNA) and translation product (protein) of these genes.

These results revealed that ribosome inactivation via the ricin A chain effectively stabilizes complexes between the transcripts and translation products of these genes. Inserting a spacer peptide into the translation product to avoid steric hindrance was effective in ribosome inactivation.

Complexes prepared using the above-described DNA constructs, which comprise either the DHFR gene, the GST gene, or the streptavidin gene, bound selectively to the MTX ligand, glutathione-Sepharose, or biotin-agarose respectively. This finding suggests that by using a molecule capable of interacting with the phenotype of a particular gene as a target material, a functional protein that binds to this target material, and an mRNA encoding that protein, can be screened from complexes formed using the above-described method. The method of the present invention enables efficient selection of functional proteins and the mRNAs that encode those proteins, without reducing the variety of peptide amino acid sequences to be screened. In addition, a complex comprising transcript and translation product can be stabilized using a genetic engineering technique, namely, the fusion of a DNA encoding the transcript and translation product with a DNA that contributes to stabilization. Thus, a further merit of this method lies in its simple procedure and selection steps free from complicated chemical synthesis.

Specifically, the present invention relates to:
[1] a DNA construct comprising the DNAs of (i) and (ii), wherein the DNAs are bound so as to express the fused transcript and translation product:
   (i) a DNA encoding an arbitrary polypeptide; and
   (ii) a DNA encoding a polypeptide having the function of stabilizing a complex comprising the transcript and translation product of the DNA defined in (i);
[2] the DNA construct according to [1], wherein the DNA defined in (ii) encodes a polypeptide having a ribozyme-inactivating function;
[3] the DNA construct according to [2], wherein a DNA encoding a spacer peptide is linked downstream of the DNA encoding a polypeptide having the ribozyme-inactivating function;
[4] the DNA construct according to [2], wherein a DNA encoding a linker peptide is inserted between the DNA encoding the arbitrary polypeptide and the DNA encoding the polypeptide having the ribozyme-inactivating function;
[5] the DNA construct according to [1] wherein the DNA defined in (ii) is a combination of a DNA encoding an RNA binding protein, and a DNA encoding an RNA to which the RNA binding protein binds;
[6] the DNA construct according to [5], wherein the fused transcript of the DNAs defined in (i) and (ii) is modified so as to lack the termination codon;
[7] the DNA construct according to [5], wherein a plurality of DNAs encoding RNA binding proteins are tandemly arranged and wherein a plurality of DNAs encoding RNAs to which the RNA binding proteins bind are tandemly arranged;
[8] the nucleic acid construct according to [7], wherein the plurality of tandemly arranged DNAs encoding RNA binding proteins are DNAs encoding a plurality of different RNA binding proteins, and wherein the plurality of tandemly arranged DNAs encoding RNAs to which the RNA binding proteins bind, are DNAs encoding RNAs to which a plurality of different RNA binding proteins bind;
[9] a nucleic acid construct comprising a complex of an mRNA defined in (i) and a DNA defined in (ii):
   (i) a fusion mRNA of an RNA encoding a DNA binding protein, and an RNA encoding an arbitrary polypeptide; and
   (ii) a DNA comprising a DNA region to which the translation product of the fusion mRNA defined in (i) binds;
[10] the nucleic acid construct according to [9], wherein the mRNA defined in (i) is hybridized with the DNA defined in (ii) to form a complex;
[11] the nucleic acid construct according to [9], wherein the 3'-end region of the mRNA defined in (i) is hybridized with the 3'-end region of the DNA defined in (ii);
[12] a DNA construct defined in the following (i) or (ii) , which is used for preparing the DNA construct as set forth in [1]:
   (i) a DNA construct comprising a DNA having a function of stabilizing a complex of the transcript and translation product of a DNA encoding an arbitrary polypeptide; and
   (ii) a DNA construct comprising a DNA having a function of stabilizing a complex of the transcript and translation product of a DNA encoding an arbitrary polypeptide, and a cloning site for the DNA encoding that arbitrary polypeptide;
[13] a method for producing a complex comprising the transcript and translation product of the DNAs defined in (i) and (ii) in the DNA construct as set forth in [1], wherein the method comprises expressing the DNAs defined in (i) and (ii);
[14] a method for producing a complex of the translation product obtained by translating the mRNA defined in (i) in the nucleic acid construct as set forth in [9], and the nucleic acid construct as set forth in [9], wherein the method comprises translating the mRNA defined in (i) and binding that translation product to the DNA defined in (ii), in the nucleic acid construct as set forth in [9];
[15] the method according to [14], wherein the method uses a nucleic acid construct in which a complex is formed by hybridizing the mRNA defined in (i) and the DNA defined in (ii);
[16] the method according to [14], wherein the method uses a nucleic acid construct in which the 3'-end region of the mRNA defined in (i) is hybridized with the 3'-end region of the DNA defined in (ii);
[17] a method for elongating the 3'-end region of the DNA defined in (ii) in a complex produced by the method as set forth in [15], wherein the method comprises contacting the complex with reverse transcriptase and carrying out a reverse-transcription reaction using the mRNA defined in (i) as a template;
[18] a method for elongating the DNA defined in (ii) in a complex produced by the method as set forth in [16], wherein the method comprises contacting the complex with reverse transcriptase and carrying out reverse-transcription reaction using the mRNA defined in (i) as a template and the DNA defined in (ii) as a primer;
[19] the method according to any one of [13] to [18], wherein the method is used in a cell-free system;
[20] A complex produced by the method as set forth in [13];
[21] a complex produced by the method as set forth in any one of [14] to [18];
[22] a method of screening for a polypeptide binding to a particular target substance, or for an mRNA encoding that polypeptide, wherein the method comprises the steps of:
   (a) forming a complex comprising the transcript and translation product of the DNAs defined in (i) and (ii) in the DNA construct as set forth in [1], by expressing the DNAs defined in (i) and (ii);
   (b) contacting the particular target substance with the complex formed in step (a); and
   (c) collecting the complex bound to that target substance;
[23] a method of screening for a polypeptide binding to a particular target substance, or for an mRNA encoding that polypeptide, wherein the method comprises the steps of:
   (a) translating the mRNA defined in (i) in the nucleic acid construct as set forth in [9],
   (a') forming a complex comprising this translation product and the nucleic acid construct as set forth in [9], by binding the translation product to the DNA defined in (ii), in the nucleic acid construct as set forth in [9];
   (b) contacting the particular target substance with the complex formed in step (a); and
   (c) collecting the complex bound to that target substance;
[24] a method of screening for a polypeptide binding to a particular target substance or for an mRNA or a DNA encoding the polypeptide, wherein the method comprises the steps of:
   (a) translating the mRNA defined in (i) in the nucleic acid construct as set forth in [10],
   (a') forming a complex comprising that translation product and the nucleic acid construct as set forth in [10], by binding the translation product to the DNA defined in (ii), in the nucleic acid construct as set forth in [10];
   (b) elongating the DNA defined in (ii) in the complex formed in step (a), by contacting the complex with reverse transcriptase and carrying out a reverse-transcription reaction using, as a template, the mRNA defined in (i) in the complex;
   (c) contacting the particular target substance with the complex formed in step (b); and
   (d) collecting the complex bound to that target substance]
[25] a method of screening for a polypeptide binding to a particular target substance or for an mRNA or DNA encoding that polypeptide, wherein the method comprises the steps of:
   (a) translating the mRNA defined in (i) in the nucleic acid construct as set forth in [11],
   (a') forming a complex comprising the translation product and the nucleic acid construct as set forth in [11], by binding the translation product to the DNA defined in (ii) in the nucleic acid construct as set forth in [11];
   (b) elongating the 3'-end region of the DNA defined in (ii) in the complex formed in step (a), by contacting the complex with reverse transcriptase and carrying out a reverse-transcription reaction using as a template the mRNA defined in (i) in the complex, and as a primer the DNA defined in (ii) in the complex;
   (c) contacting the particular target substance with the complex formed in step (b); and
   (d) collecting the complex bound to that target substance;
[26] a method of screening for a polypeptide binding to a particular target substance or for an mRNA or DNA encoding that polypeptide, wherein the method comprises the steps of:
   (a) translating the mRNA defined in (i) in the nucleic acid construct as set forth in [10],
   (a') forming a complex comprising the translation product and the nucleic acid construct as set forth in [10], by binding the translation product to the DNA defined in (ii) in the nucleic acid construct as set forth in [10];
   (b) contacting the particular target substance with the complex formed in step (a);
   (c) elongating the DNA defined in (ii) in the complex bound to the target substance, by contacting the complex with reverse transcriptase and carrying out reverse-transcription reaction using, as template, the mRNA defined in (i) in the complex; and
   (d) collecting the complex bound to the target substance;
[27] a method of screening for a polypeptide binding to a particular target substance, or for an mRNA or a DNA encoding that polypeptide, wherein the method comprises the steps of:
   (a) translating the mRNA defined in (i) in the nucleic acid construct as set forth in [11],
   (a') forming a complex comprising the translation product and the nucleic acid construct as set forth in [11], by binding the translation product to the DNA defined in (ii) in the nucleic acid construct as set forth in [11];
   (b) contacting the particular target substance with the complex formed in step (a);
   (c) elongating the 3'-end region of the DNA defined in (ii) in the complex bound to the target substance, by contacting the complex with reverse transcriptase and carrying out a reverse-transcription reaction using, as a template, the mRNA defined in (i) in the complex, and, as a primer, the DNA defined in (ii) in the complex; and
   (d) collecting the complex bound to the target substance;
[28] the method according to any one of [22] to [27], wherein the target substance is fixed on a carrier;
[29] a kit used for the screening method as set forth in [21], wherein the kit comprises the DNA construct as set forth in [1] or [12], or the complex as set forth in [20]; and
[30] a kit used for the screening method as set forth in any one of [23] to [27], wherein the kit comprises the nucleic acid construct as set forth in [9], or the complex as set forth in [21].

The methods and embodiments of the present invention are illustrated in detail below.

### (1) Nucleic acid constructs

The DNA constructs of the present invention include: (i) DNA encoding an arbitrary polypeptide; and (ii) DNA comprising the function of stabilizing a complex between the transcript and translation product of that DNA. In this DNA construct, the DNAs of (i) and (ii) are linked together so as to ensure expression of the fused transcript and translation product.

The term "DNA encoding an arbitrary polypeptide" refers to the desired DNA for which a complex between transcript and translation product will be formed. In screening for functional proteins, for example, a cDNA library or random library can be preferably used.

There is no limitation as to the type of DNA comprising the function of stabilizing the complex between transcript and translation product. The first example of such preferred a DNA is a DNA encoding a polypeptide comprising the function of inactivating a ribosome.

As used herein, the phrase "a polypeptide comprising the function of inactivating a ribosome" means not only a polypeptide that disrupts and thus inactivates a ribosome, but also a polypeptide that can stop ribosome movement along an mRNA chain, and thus inhibit the translational function of that ribosome.

Such polypeptides include N-glycosylases and RNases. Specific examples of N-glycosylases include pokeweed antiviral protein (PAP) (*Phytolacca americana*), momordin (*Momordica charantia*), luffin (*Luffa cylindrica*), bryodin (*Bryonia dioica*), dianthin (*Dianthus caryophyllus*), trichosanthin (*Trichosanthes anguina*), a-momorcharin (*Momordia charantia*), saporin (*Saponaria officinalis*), ricin A-chain (RTA) (*Ricinus communis*), abrin A-chain (*Abrus precatorius*), mistletoe lectin I (MLI) (*Viscum album*), shiga toxin AI, and diphtheria toxin (DT) A-chain. A specific example of RNases is α-sarcin.

Ricin is described in the reference: "The RNA N-glycosidase activity of ricin A-chain. The characteristics of the enzymatic activity of ricin A-chain with ribosomes and with rRNA", J. Biol. Chem., 1988 Jun 25; 263(18):8735-9. α-sarcin is described in the reference: "The ribonuclease activity of the cytotoxin alpha-sarcin. The characteristics of the enzymatic activity of alpha-sarcin with ribosomes and ribonucleic acids as substrates", J. Biol. Chem. 1983 Feb 25, 258(4):2662-7. The pokeweed antiviral protein (PAP) is described in the reference: "X-ray crystallographic analysis of the structural basis for the interaction of pokeweed antiviral protein with guanine residues of ribosomal RNA", Protein Sci., 1999 Nov., 8(11):2399-405.

In an embodiment of the preferred DNA construct of the present invention, a DNA encoding a spacer peptide is ligated downstream of a DNA encoding a polypeptide comprising a ribosome-inactivating function. This "spacer peptide" allows the polypeptide comprising the ribosome-inactivating function to attack the ribosome in a cis-acting manner, prior to termination of translation by that ribosome. It is preferable that the spacer peptide has an unfolded structure. The spacer peptide comprises a chain length such that, upon translation, the polypeptide comprising the ribosome-inactivating function can attack the ribosome in a cis-acting manner. The chain length typically ranges from 50 to 200 amino acids, preferably from 100 to 140 amino acids.

In an alternative embodiment of the preferred DNA construct of the present invention, a DNA encoding a linker peptide is inserted between a DNA encoding an arbitrary polypeptide in the DNA construct of the present invention, and a DNA encoding a polypeptide comprising the function of inactivating a ribosome. This "linker peptide" refers to a polypeptide inserted between the polypeptides fused in the translation product (the fusion protein) of a DNA construct of the present invention, in order to avoid folding inhibition by steric hindrance of the polypeptides to be fused. There is no limitation as to the chain length of the linker peptide. Typically, peptides ranging from about 10 amino acids to about 30 amino acids are used.

The second example of a preferred DNA comprising the function of stabilizing the complex between transcript and translation product is a combination of a DNA encoding an RNA-binding protein, and a DNA encoding an RNA to which that RNA-binding protein binds. When using this combination of a DNA sequence encoding an RNA-binding protein and a DNA sequence encoding an RNA to which that RNA-binding protein binds, it is preferable that the DNA construct contains a plurality of tandemly arranged DNAs encoding RNA-binding proteins, and a plurality of tandemly arranged DNAs encoding RNAs to which those RNA-binding proteins bind. Such an arrangement can strengthen the interaction between the RNA-binding protein and the RNA.

More preferably, DNAs that each encode different RNA-binding protein can be used as the DNAs encoding several RNA-binding proteins. Furthermore, as DNAs encoding RNAs to which the above-mentioned several RNA-binding proteins bind, DNAs that each encode a different RNA to which the above-described several different RNA-binding proteins bind, are used as the DNAs encoding RNAs to which the variety of RNA-binding proteins bind. The several different DNAs as described above can be used to prevent the formation of network structures among different complexes.

Combinations of RNA-binding proteins and RNAs to which those RNA-binding proteins bind include the combination of Tat protein and TAR RNA or Tat aptamer; the combination of Rev protein and RRE RNA or Rev aptamer; the combination of U1A spliceosomal protein and U1A spliceosomal protein aptamer or U1A Sn RNA; and the combination of a zinc finger protein or a mutant thereof and RNA recognized by that protein. In addition, the combination of MS2 coat protein and an RNA sequence comprising a specific binding motif corresponding to that protein can be used in the present invention.

The Tat-TAR or Tat aptamer is referred to as "A novel RNA motif that binds efficiently and specifically to the Tat protein of HIV and inhibits the trans-activation by Tat of transcription *in vitro* and *in vivo*", Genes Cells., 2000 May. 5(5):371-88. The Rev-RRE complex is referred to as: "A structural model for the HIV-1 Rev-RRE complex deduced from altered-specificity rev variants isolated by a rapid genetic strategy", Cell. 1996 Oct 4;87(1):115-25. The U1A spliceosomal protein is referred to as: "Crystal structure at 1.92 A resolution of the RNA-binding domain of the U1A spliceosomal protein complexed with an RNA hairpin", Nature., 1994 Dec 1;372 (6505) :432-8.

In addition, documents describing the interaction between zinc-finger proteins and RNA include: Friesen, W. J. *et al*. "Specific RNA binding proteins constructed from zinc fingers", Nat. Struct. Biol., 1998 Jul. 5(7):543-6; McColl, D. J. *et al*., "Structure-based design of an RNA-binding zinc finger", Proc. Natl. Acad. Sci. USA., 1999 Aug 17, 96(17):9521-6; Friesen, W. J. *et al*., "Phage display of RNA binding zinc fingers from transcription factor IIIA", J. Biol. Chem., 1997 Apr 25, 272(17) :10994-7; Theunissen, O. *et al*., "RNA and DNA binding zinc fingers in Xenopus TFIIIA", Cell, 1992 Nov 13, 71 (4) : 679-90; Clemens, K. R. *et al*., "Molecular basis for specific recognition of both RNA and DNA by a zinc finger protein", Science, 1993 Apr 23, 260 (5107) :530-3; and Joho, K. E. *et al*., "A finger protein structurally similar to TFIIIA that binds exclusively to 5S RNA in Xenopus", Cell, 1990 Apr 20;61(2):293-300.

In a preferred embodiment of the DNA construct of the present invention, the DNA construct has been modified such that the fused transcript expressed from the DNA construct lacks a termination codon. This results in delay of ribosome release from the complex comprising the transcript and translation product, and this delay can stabilize the complex.

The above-described DNA constructs of the present invention can be prepared, for example, by inserting a DNA comprising the function of stabilizing a complex between transcript and translation product, into a vector which comprises a DNA encoding an arbitrary polypeptide. The DNA constructs can also be prepared by inserting a DNA encoding an arbitrary polypeptide into the cloning site of a vector which comprises i) a DNA comprising the function of stabilizing a complex between transcript and translation product, and ii) the cloning site of the DNA encoding that arbitrary polypeptide.

For use in the preparation of the above-described DNA constructs, the present invention also provides: (i) a DNA construct that comprises a DNA having the function of stabilizing a complex between the transcript and translation product of a DNA encoding an arbitrary polypeptide, and (ii) a DNA construct that comprises a DNA having the function of stabilizing a complex between the transcript and translation product of a DNA encoding an arbitrary polypeptide, and a cloning site for the DNA encoding that arbitrary polypeptide.

In the present invention, the nucleic acid construct comprising mRNA and DNA is a complex of: (i) a fusion mRNA between an RNA encoding a DNA-binding protein and an RNA encoding an arbitrary polypeptide, and (ii) a DNA comprising a DNA region to which the translation product of the fusion mRNA of (i) binds. In the nucleic acid construct, the mRNA of (i) and the DNA of (ii) preferably form a complex by hybridization (hydrogen bonds). There is no limitation as to the number of base pairs participating in the hybridization, as long as the mRNA of (i) and the DNA of (ii) in the nucleic acid construct stably bind with each other, however this number is preferably ten or more base pairs.

A DNA corresponding to the mRNA of (i) in the nucleic acid construct can be synthesized by carrying out reverse transcription using this mRNA as a template. In addition to the transcript and translation product, DNAs encoding these transcript and translation product can be used as components of the complex.

When the nucleic acid construct is prepared such that the 3'-end region of the mRNA of (i) hybridizes with the 3'-end region of the DNA of (ii), reverse transcription can be carried out using the mRNA of (i) as a template, and the DNA of (ii) as a primer.

When an alternative reverse transcription primer is used instead of the DNA of (ii), it is not essential that the 3'-end regions of the mRNA of (i) and the DNA of (ii) hybridize with each other in the nucleic acid construct. Hybridization of the 5'-end regions with each other will suffice. In this case, it is necessary to add a primer (an oligonucleotide hybridizing specifically to the mRNA of (i)) to the reaction system to achieve reverse transcription.

See the reference of FEBS Lett., 2001 Nov 23, 508 (3) :309-12 for mRNA/DNA binding used to prepare the present invention's nucleic acid construct comprising mRNA and DNA.

### (2) Methods for producing a complex between transcript and translation product

The present invention also provides methods for producing the above-described complex between transcript and translation product. An embodiment of the production methods comprises expressing a fusion DNA which comprises: (i) a DNA encoding an arbitrary polypeptide and (ii) a DNA comprising the function of stabilizing a complex between the transcript and translation product of that DNA, by adding or introducing the above-described DNA construct into an appropriate transcription/translation system. The transcript and translation product produced by expressing the fusion DNA can form a stable complex through the ribosome-inactivating activity of the polypeptide in the translation product, or through interaction between the RNA-binding protein in the translation product and the binding site in the transcript.

In an alternative embodiment, the production method comprises translating the mRNA of (i) in the nucleic acid construct, and allowing the translation product to bind to the DNA of (ii) in the nucleic acid construct, by adding or introducing the nucleic acid construct that comprises the above-described RNA and DNA into an appropriate transcription/translation system. A stable complex is formed through the interaction of the DNA-binding protein in the translation product and the DNA of (ii) in the nucleic acid construct. DNA corresponding to the mRNA can be synthesized by conducting reverse transcription, using as a template the mRNA of (i) in the complex to elongate the DNA of (ii). When using a nucleic acid construct in which the 3'-end region of the mRNA of (i) hybridizes with the 3'-end region of the DNA of (ii), reverse transcription can be conducted using the DNA of (ii) as a primer.

Transcription/translation systems that can be used in this method include cell-free transcription/translation systems, and living cells. There is no limitation as to the type of cell-free transcription/translation system or living cell, as long as it ensures the expression of the transcript and translation product from the nucleic acid construct (the expression of the translation product when using a nucleic acid construct comprising mRNA and DNA) when the nucleic acid construct of the present invention is added or introduced into it. In this method, a cell-free transcription/translation system can be preferably used, and a transcription/translation system comprising cell extracts is particularly preferable.

Cell-free transcription/translation systems include those comprising extracts of prokaryotic or eukaryotic cells, for example, extracts of *E. coli* cells, rabbit reticulocytes, and wheat germ. In addition, translation systems using living cells include prokaryotic and eukaryotic cells, for example, *E. coli* cells.

A complex obtained by this method and comprising transcript and translation product is also included in the present invention. Such a complex can exist stably, and as described below, can be used to screen for a polypeptide that interacts with a particular target material, or for the mRNA or DNA encoding that polypeptide.

### (3) Method of screening for a polypeptide that binds to a particular target material, or for the mRNA or a DNA encoding that polypeptide

The present invention also provides a method of screening for a polypeptide that binds to a particular target material, or for an mRNA or DNA encoding that polypeptide.

In the first embodiment of the methods of the present invention, a DNA construct of the present invention is used. In this embodiment, a fusion DNA which comprises: (i) a DNA encoding an arbitrary polypeptide; and (ii) a DNA having the function of stabilizing the complex between the transcript and translation product derived from the above-described DNA construct, is first expressed, and a complex is then formed between the fusion transcript and translation product derived from the DNA (step (a)). A particular target material is then contacted with the complex formed in step (a) (step (b)), and the complex bound to that target material is then recovered (step (c)).

In the second to fourth embodiments of methods of the present invention, a nucleic acid construct is used, which comprises an mRNA and a DNA of the present invention.

In the second embodiment, the methods do not comprise the step of reverse transcription. In the second embodiment, the translation product is first allowed to bind to the DNA of (ii) in the nucleic acid construct, by translating the mRNA of (i) in the nucleic acid construct that comprises the mRNA and DNA of the present invention. A complex is thus formed between the translation product and the nucleic acid construct (step (a)). A particular target material is then contacted with the complex formed in step (a) (step (b)), and the complex bound to the target material is then recovered (step (c)).

In the third and fourth embodiments, the methods of the present invention comprise the step of carrying out reverse transcription: In the third embodiment, reverse transcription is conducted prior to binding between a target material and a complex. In the fourth embodiment, reverse transcription is conducted after binding between a target material and a complex.

In the third embodiment of the methods of the present invention, the translation product is first allowed to bind to the DNA of (ii) in the nucleic acid construct, by translating the mRNA of (i) in the nucleic acid construct that comprises the mRNA and DNA of the present invention. A complex is thus formed between the translation product and the nucleic acid construct (step (a)). The complex formed in step (a) is then contacted with reverse transcriptase to carry out reverse transcription, using as a template the mRNA of (i) in the complex to elongate the DNA of (ii) in the complex (the step (b)). The particular target material is then contacted with the complex formed in step (b) (the step (c)), and the complex bound to the target material is then recovered (step (d)).

In the fourth embodiment of the method of the present invention, the translation product is allowed to bind to the DNA of (ii) in the nucleic acid construct, by translating the mRNA of (i) in the nucleic acid construct that comprises the mRNA and DNA of the present invention. In this way a complex is formed between the translation product and the nucleic acid construct (step (a)). The particular target material is then contacted with the complex formed in step (a) (step (b)). The complex bound to the target material is then contacted with reverse transcriptase to conduct reverse transcription, using as a template the mRNA of (i) in the complex to elongate the DNA of (ii) in the complex (step (c)). The complex bound to the target material is then recovered (step (d)).

In the third and fourth embodiments of the methods of the present invention, when using a nucleic acid construct in which the 3'-end region of the mRNA of (i) hybridizes with the 3'-end region of the DNA of (ii), the reverse transcription can be simply carried out by using the DNA of (ii) as a primer.

There is no limitation as to the type of target materials in the methods of the present invention, and various target materials can be used depending on the reason for screening. Specifically, target materials include peptides, proteins, low-molecular-weight compounds, ligands, enzymes, antibodies, and environmental pollutants. For example, when a ligand is used as a target material, a receptor that binds with that ligand can be screened. Such screening is useful in identifying ligands for orphan receptors. For the convenience of complex recovery, it is preferable to immobilize target materials on carriers. Alternatively, and depending on the purpose, screening may be carried out using a support on which a plurality of target materials has been arranged.

The screening of the present invention is exemplified by the method described below. After transcription/translation of the nucleic acid construct of the present invention (after translation, when using a nucleic acid construct comprising mRNA and DNA), a suspension of a target material bound to an immobilized solid carrier such as agarose is added, kept for a certain period of time to allow the binding of the target material with the product from the gene of interest. After removing the supernatant containing unbound mRNA and protein from the mixture, the solid carrier is washed with an appropriate buffer. The mRNA (or DNA) bound to the solid carrier can be isolated by elution at a high temperature using a solution containing urea.

The present invention provides kits to be used in the screening of the present invention. Screening kits using DNA constructs of the present invention can comprise any one or multiple samples of: for example, (I) a DNA construct of the present.invention; (II) the following (i) or (ii) for use in preparing the DNA construct of the present invention: (i) a DNA construct comprising a DNA which comprises the function of stabilizing a complex between the transcript and translation product derived from a DNA encoding an arbitrary polypeptide, or (ii) a DNA construct comprising a DNA which comprises the function of stabilizing a complex between the transcript and translation- product derived from a DNA encoding an arbitrary polypeptide, and a cloning site of the DNA encoding that arbitrary polypeptide; and (III) a complex formed through expression of a DNA construct of the present invention.

Alternatively, the screening kit using a DNA construct of the present invention comprising an mRNA and a DNA of the present invention can comprise either or both samples: for example, (I) a nucleic acid construct that comprises an mRNA and a DNA of the present invention; and (II) a complex formed through expression of a nucleic acid construct that comprises an mRNA and a DNA of the present invention (which also comprises the DNA synthesized from the mRNA in the complex by reverse transcription).

### Brief Description of the Drawings

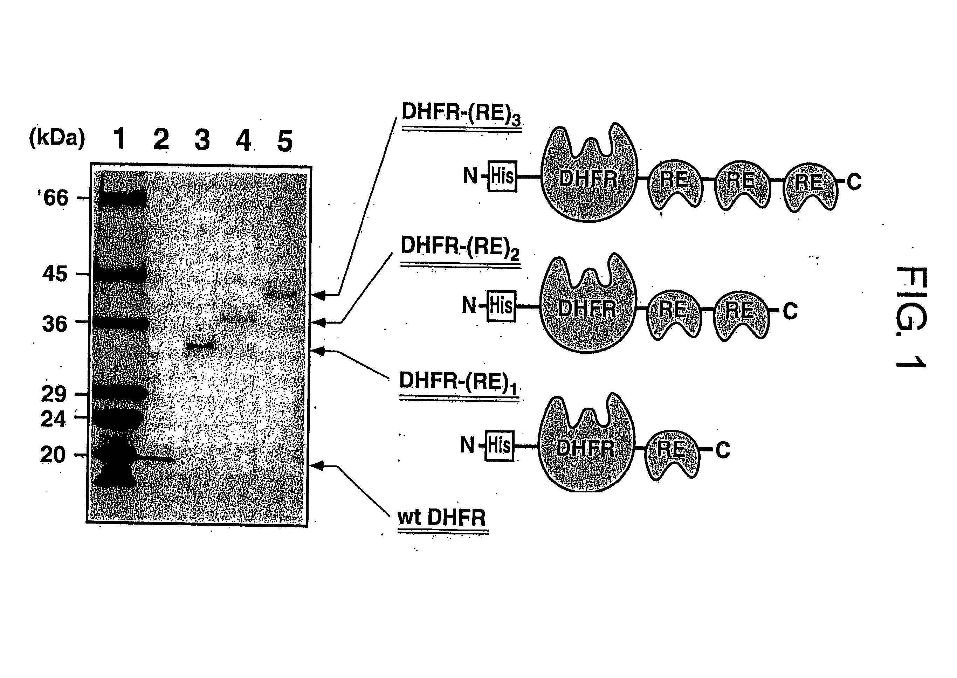

Fig. 1 depicts a schematic illustration of the purified DHFR-(RE)₁₋₃ fusion proteins and wild type (wt) DHFR, and a photograph showing the gel after sodium dodecyl sulfate-polyacrylamide gel electrophoresis (SDS-PAGE). The purified recombinant proteins (about 100 µg) were fractionated with SDS-PAGE, and visualized using Coomassie Brilliant Blue staining.
   Lane 1, marker;
   lane 2, wild type DHFR;
   lane 3, DHFR-(RE)₁;
   lane 4, DHFR-(RE)₂; and
   lane 5, DHFR-(RE)₃.
   The molecular weights of the marker proteins are indicated to the left. A poly-glycine linker was inserted between DHFR and the first RE peptide, and between RE peptides neighboring each other. A stretch of poly-histidine (His) was attached to the N-terminus of DHFR for. the convenience of purification.

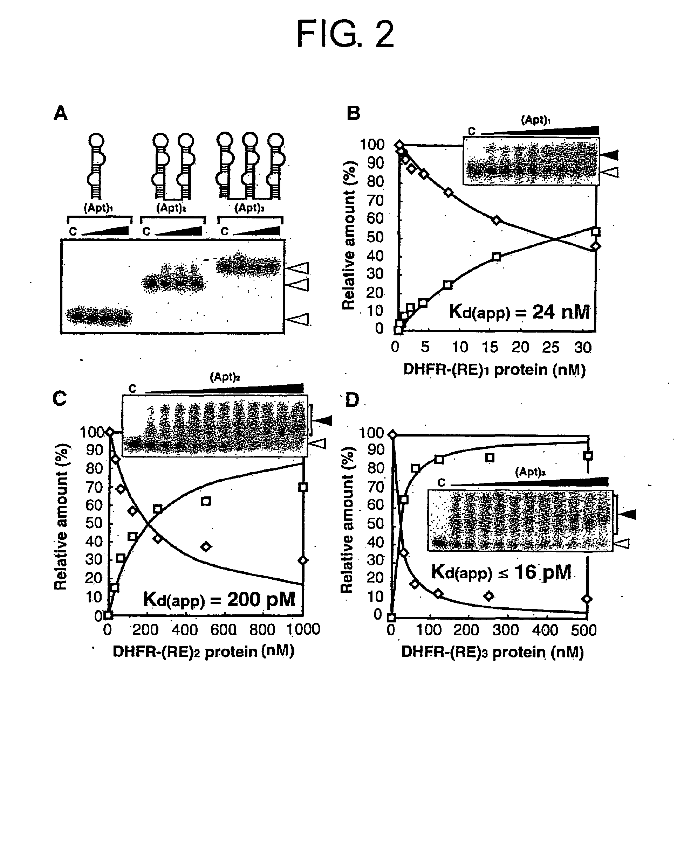

Fig. 2 depicts diagrams and photographs showing experimental results for the dissociation constant (K_{d}). Lane C in each panel contains only the aptamer. Closed arrows indicate protein-RNA complexes, and open arrows indicate free RNAs. ³²P radioactivity was used to determine relative amounts of complex and free RNA. Equilibrium dissociation constants were determined from the theoretical curves provided by the least square method, in which the concentration of free RNA or complex is plotted against peptide concentration. Diamonds indicate the relative amounts of free RNA, and squares indicate the relative amounts of the complex. The closed narrow triangles in each panel indicate an increase in protein concentration.
   (A) is a photograph showing the loss of interaction between (Apt)₁₋₃ and wild-type DHFR. Lanes C contain 1 nM (Apt)₁₋₃ with wild-type DHFR added at concentrations of 20, 100, and 500 nM respectively, as indicated by the narrow triangles.
   (B) depicts a diagram and photograph showing binding between (Apt)₁ and DHFR-(RE)₁. DHFR-(RE)₁ was mixed at concentrations of 0.25, 0.5, 1, 2, 4, 8, 16, or 32 nM with 30 pM (Apt)₁ (lane C), as indicated by the narrow triangles.
   (C and D) depict diagrams and photographs showing binding between (Apt)₂₋₃ and DHFR-(RE)₂₋₃ protein. The protein was mixed at a concentration of 0.03, 0.06, 0.12, 0.25, 0.5, 1, 2, 4, 8, or 16 nM with 30 pM (Apt)₂ or (Apt)₃ RNA (lane C) , as indicated by the narrow triangles.

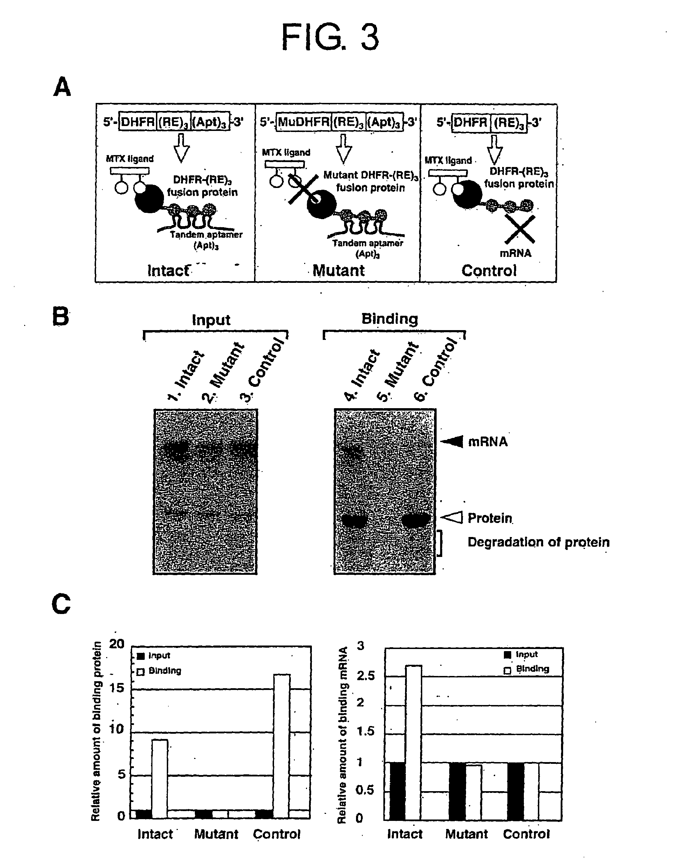

Fig. 3 depicts diagrams and photographs showing models for the selection of functional proteins using the interaction between (Apt)₃ and (RE)₃ in vitro.
   (A) is a schematic illustration showing the interaction between translated proteins, transcribed RNAs, and methotrexate-immobilized agarose beads (MTX ligand).
   (B) depicts photographs showing the results of SDS-PAGE for DHFR- (RE)₃ protein and mRNA (wild type) enriched by MTX-beads. Each sample was fractionated using 15% SDS-PAGE. After the translation reaction, 1 µl of cell lysate was loaded onto each of lanes 1 to 3. 10 µl of elution solution containing mRNA and protein bound to MTX-beads was loaded onto each of lanes 4 to 6. Lanes 1 and 4 contain the wild-type protein. Lanes 2 and 5 contain the mutant protein. Lanes 3 and 6 contain a control sample.
   (C) consists of diagrams showing the results of quantitation of the protein and mRNA indicated in (B).

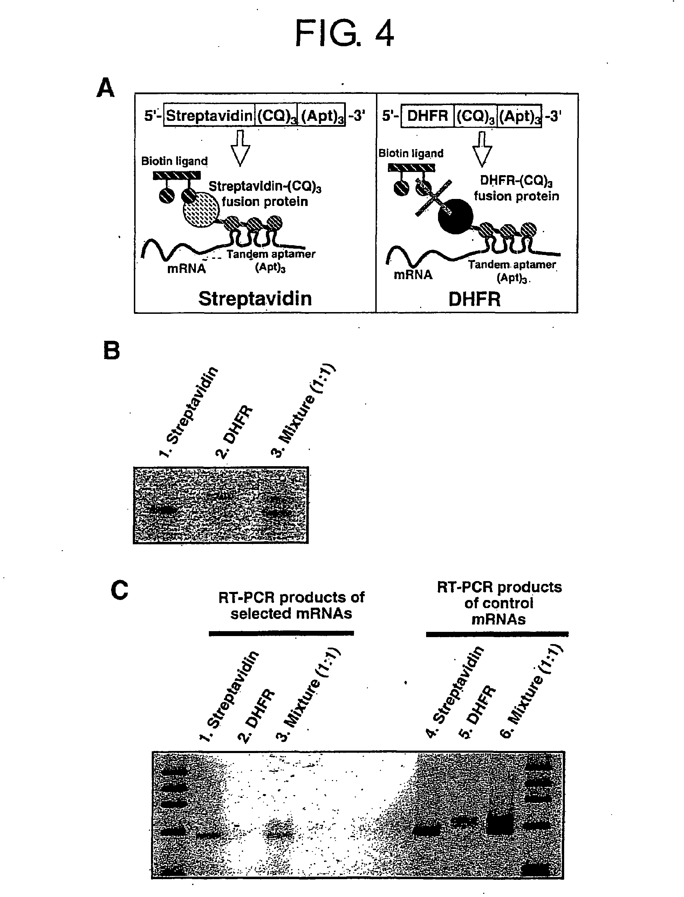

Fig. 4 depicts diagrams and photographs showing *in-vitro* selection of streptavidin-encoding mRNA from the pool of mixed streptavidin and DHFR mRNAs.
   (A) is a schematic illustration showing the interaction between transcribed RNAs, translated proteins, and biotin-immobilized agarose beads (biotin ligand).
   (B) is a photograph showing the results of denaturing PAGE on streptavidin - (RE)₃-(Apt)₃ mRNA enriched with biotin agarose beads. After translation and subsequent selection, each sample was fractionated using 4% denaturing PAGE.

   Lane 1: isolated streptavidin-(CQ)₃-(Apt)₃ mRNA. mRNA (1.25 µg) encoding streptavidin was translated in cell lysate, and then selected using biotin beads.
   Lane 2: isolated DHFR-(CQ)₃-(Apt)₃ mRNA. mRNA (1.25 µg) encoding DHFR was translated in cell lysate, and then selected using biotin beads. Since DHFR is not expected to interact with biotin beads, the level of bound DHFR-(CQ)₃-(Apt)₃ mRNA corresponds to the degree of non-specific (background) interaction.
   Lane 3: isolated streptavidin-(CQ)₃-(Apt)₃ and DHFR mRNA (upper band, DHFR mRNA; and lower band, streptavidin). The respective mRNAs encoding streptavidin and DHFR were mixed at a ratio of 1:1. The mixture was translated in cell lysate, and then selected using biotin. beads.

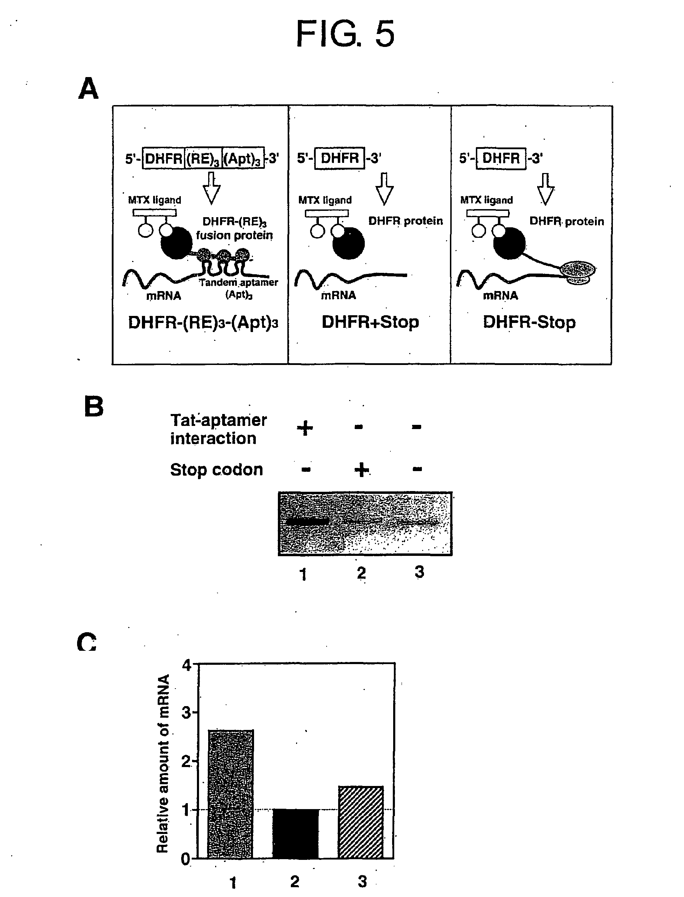

Fig. 5 depicts diagrams and a photograph showing the comparison of intensities of the interaction between (RE)₃-(Apt)₃ and ribosome-display system in the selection of functional DHFR in rabbit reticulocyte lysate.
   (A) is a schematic illustration showing the interaction among the transcribed RNAs, the translated proteins, and methotrexate-immobilized agarose beads (MTX ligand).
   (B) is a photograph showing the result of electrophoresis of RT-PCR products using 2% agarose for DHFR-(RE)₃-(Apt)₃, DHFR-Stop or DHFR+Stop mRNA enriched by MTX-beads. The PCR was carried out for 20 cycles. Each sample was fractionated using a 2% Sea Kem GTG agarose gel (FMC, Riceland, Maine USA). Lane 1, PCR product from DHFR-(RE)₃-(Apt)₃ mRNA; lane 2, PCR product from DHFR+Stop mRNA; and lane 3, PCR product from DHFR-Stop mRNA. The PCR products were analyzed·once, however, the mRNA quantitation result shown in Fig. 5C is more reliable.
   (C) is a diagram showing the result of quantitation of enriched mRNA. The relative amount of selected mRNA was determined from the radioactivity of ³²P-labeled mRNA. The respective lane numbers correspond to those shown in Fig. 5B. The result is an average of independent experiments carried out in quadruplicate.

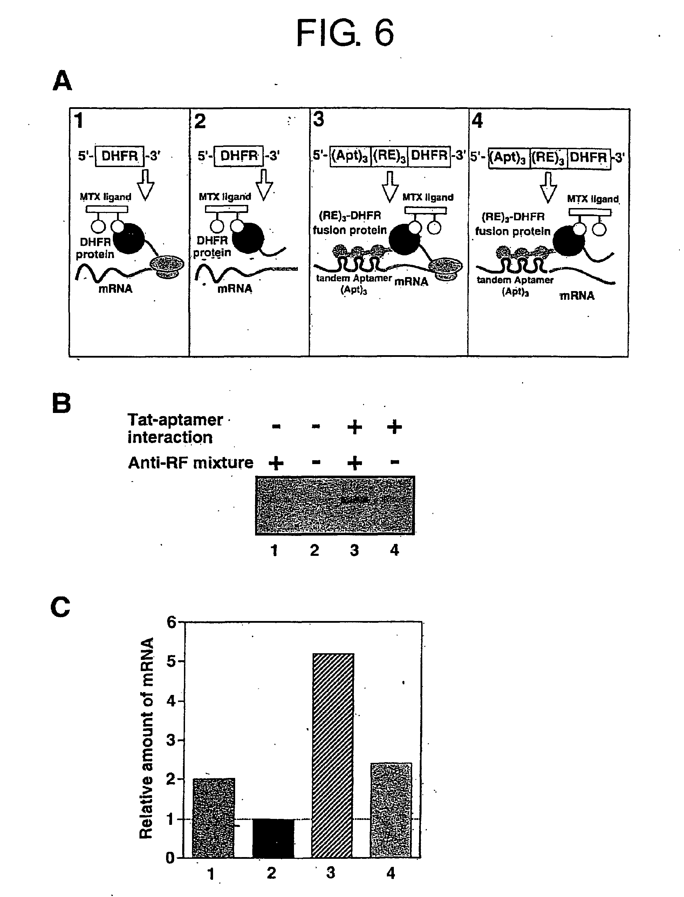

Fig. 6 depicts diagrams and a photograph showing the increased stability of the complex between each mRNA and its product, using the (RE)₃-(Apt)₃ interaction and ribosome-display system in combination, and in *E. coli* S30 strain cell extract.
   (A) is a schematic illustration for the interaction between transcribed RNAs, translated proteins, and methotrexate-immobilized agarose beads (MTX ligand).
   (B) is a photograph showing the result of electrophoresis of the RT-PCR products using 2% agarose gel in the presence (lanes 1 and 3) or absence (lanes 2 and 4) of the polyclonal antibody IgGs against the termination factors (RF1, RF2, and RF3 from E. coli), and DHFR-Stop mRNA (lanes 1 and 2) and (Apt)₃-(RE)₃-DHFR-Stop mRNA (lanes 3 and 4) enriched by MTX-beads. The selected mRNA was incubated with MTX-agarose beads under the same conditions as indicated in Fig. 5.
   (C) is a diagram showing the result of quantitation of the RT-PCR products indicated in (B). The result was obtained as an average in independent experiments carried out in duplicate.

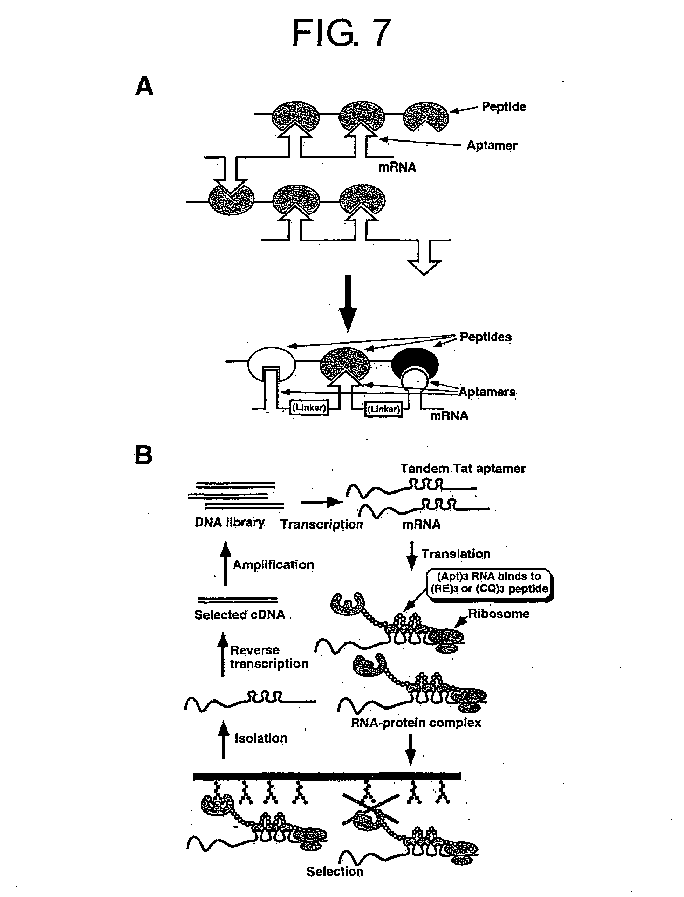

Fig. 7A depicts a diagram showing the possibility of networking due to binding between RNA and protein. In principle, the tandemly arranged Tat (RE) peptide and aptamer can bind with each other not only intramolecularly but also intermolecularly. In the case of intermolecular binding, RNA-protein binding will result in networking. This intermolecular interaction can be minimized by linking different peptides and aptamers instead of linking the same peptides and aptamers in tandem.
Fig. 7B depicts a diagram showing a schematic illustration for the first embodiment of the method for selecting functional proteins *in vitro*. The enrichment can be achieved via multiple cycles comprising production of the binding protein, transcription, translation, affinity selection, and PCR. In the step of translation, the tandemly arranged aptamer (Apt)₃ binds with tandemly arranged RE peptide (RE)₃ with exceedingly high affinity. This binding ensures binding between the protein and the mRNA. In Example 1, DHFR and streptavidin were used as targets in the selection step. However, an arbitrary protein can be used in combination with the tandemly arranged aptamer (Apt)₃ and tandemly arranged RE peptide (RE)₃.

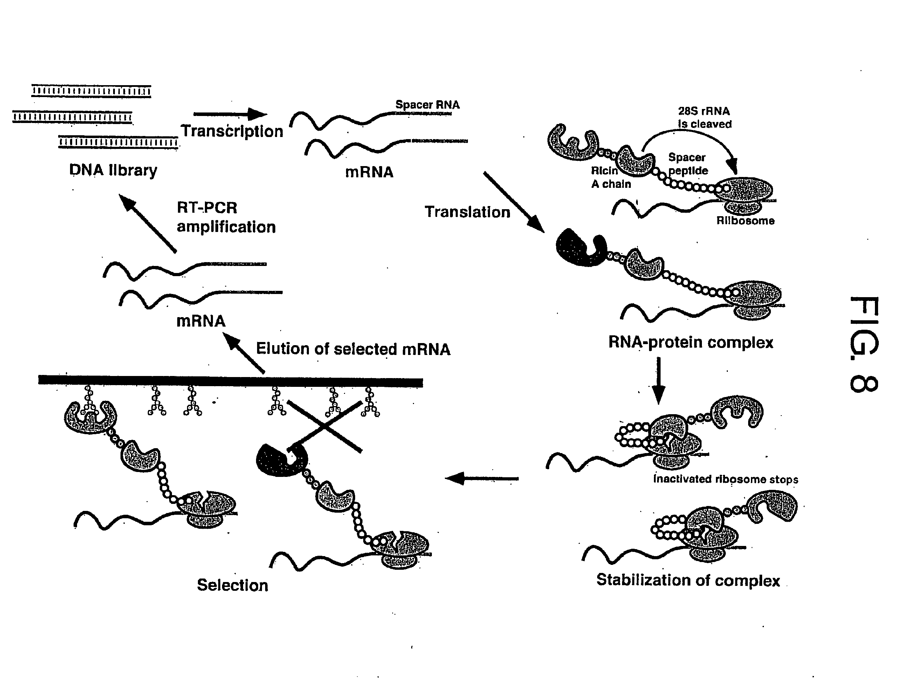

Fig. 8 depicts a diagram showing a model for an *in-vitro* protein selection system using ricin. A random DNA library or cDNA library is used. The ricin and spacer sequences are attached to the 3' end of this sequence. After transcription/translation of a protein from the library, the ricin is also translated. The ricin inactivates ribosomes by intramolecular reaction, thus terminating ribosome-mediated translation. As a result, an RNA-ribosome-protein complex is formed. The RNA in the complex encodes the genetic information on the protein, and thus selection of a particular functional protein results in recovery of an RNA comprising the genetic information of that protein. This RNA is amplified by reverse transcription/PCR, and the sequence of the selected protein can be determined by decrypting the genetic information. The above-described cycle can be repeated for higher selection pressure.

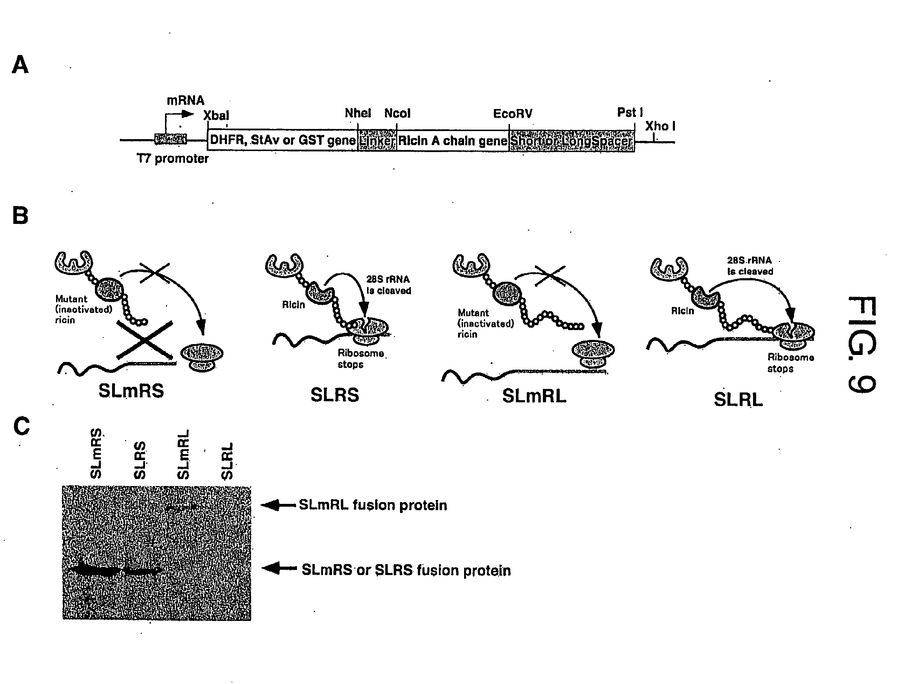

Fig. 9A depicts a diagram showing the DNA sequence used in the model of the protein selection system. Genes for the T7 promoter, streptavidin or GST sequence, the linker sequence, the ricin A chain sequence or the inactive ricin A chain sequence (prepared by introducing mutations in a portion of the ricin A chain sequence) , and the M13 Phage Gene III-derived spacer sequence (360 bp or 1212 bp) were inserted in this order from the 5'-end.. The streptavidin and GST sequences are the target proteins for selection. The linker sequence encodes a peptide linker and was inserted to prevent steric hindrance between streptavidin or GST, and the ricin A chain. The spacer sequence was inserted to prevent the ribosome from inhibiting other proteins' activities, such as folding.
Fig. 9B depicts schematic illustrations for the translation of SLmRS, SLRS, SLmRL, and SLRL genes. Ricin does not function in the SLmRS and SLmRL genes, and so the RNA-ribosome-protein complex does not form. The proteins thus dissociate from the ribosomes, and protein synthesis proceeds efficiently.
Fig. 9C depicts a photograph showing proteins translated from SLmRS, SLRS, SLmRL, and SLRL genes. The proteins were labeled with ³⁵S-methionine, and detected by SDS-PAGE using 10% gel. The shorter the linker the more protein was produced. When ricin was inactive, a larger amount of protein was produced. Protein synthesis was negligible in the case of the SLRL gene.

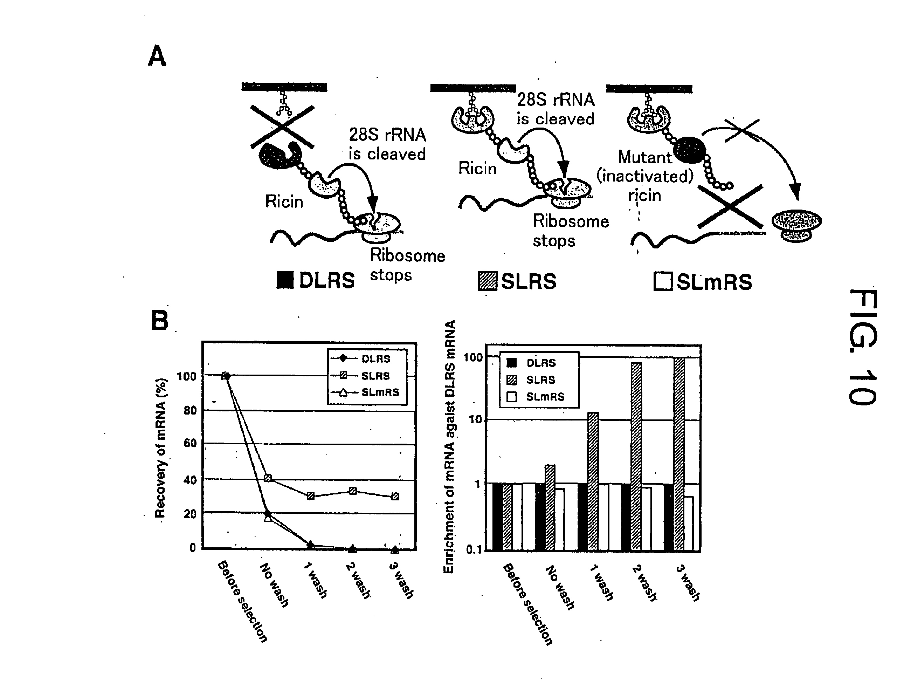

Fig. 10A is a schematic illustration of the selection of mRNA encoding streptavidin protein using biotin agarose (SLRS). mRNA encoding DHFR does not bind to biotin agarose (DLRS). In addition, when ricin is inactivated, mRNA encoding streptavidin protein does not bind to biotin agarose (SLmRS).
Fig. 10B consists of diagrams showing the amount of streptavidin protein-encoding mRNA recovered using biotin agarose. After translation, mRNA was bound to biotin agarose, unbound mRNA was washed away, and residual RNA was quantified. After three washes, 30% of the SLRS mRNA used in the translation had bound to the biotin agarose. However, after three washes, hardly any DLRS and SLmRS mRNA had bound to the agarose. After three washes, SLRS mRNA had been enriched 100 times or more.

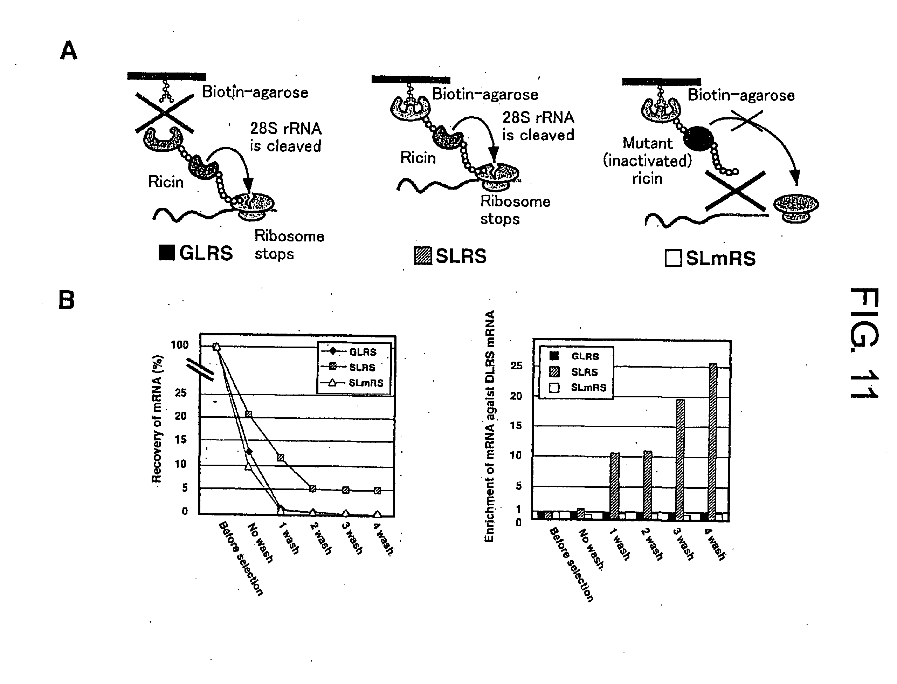

Fig. 11A is a schematic illustration for the selection of mRNA encoding streptavidin protein using biotin agarose (SLRS). GST-encoding mRNA does not bind to biotin agarose (GLRS). In addition, mRNA encoding streptavidin protein does not bind to biotin agarose (SLmRS) when ricin has been inactivated.
Fig. 11B consists of diagrams showing the amount of streptavidin protein-encoding mRNA recovered using biotin agarose. After translation, mRNA was bound with biotin agarose, unbound mRNA was washed away, and residual RNA was quantified. After four washes, 5% of SLRS mRNA used in the translation had bound to biotin agarose. However, after four washes, hardly any GLRS and SLmRS mRNA had bound to the agarose. SLRS mRNA was enriched 25 times or more after four washes.

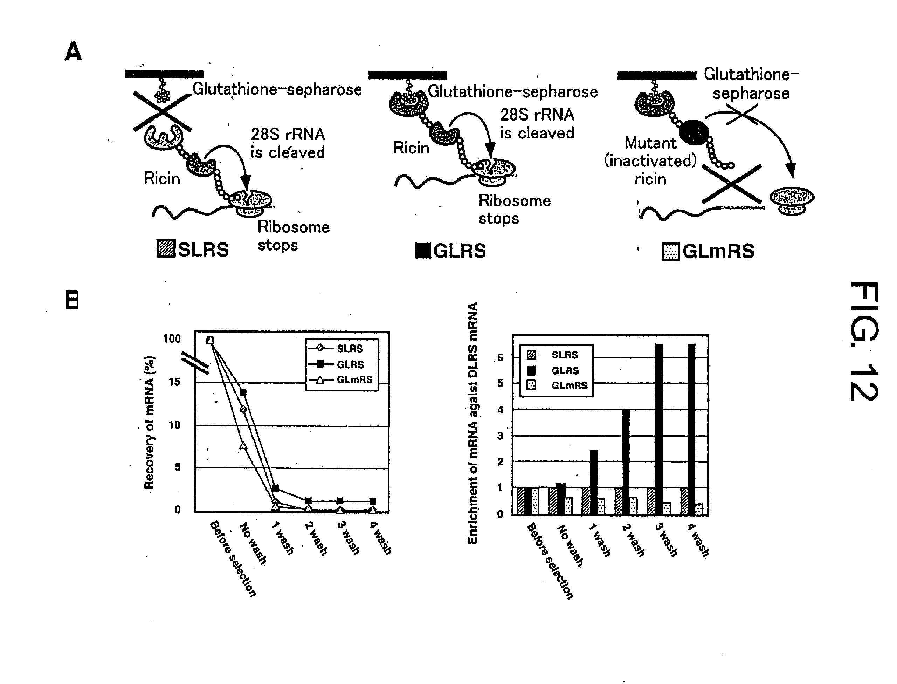

Fig. 12A is a schematic illustration for the selection of mRNA encoding GST protein by glutathione Sepharose (GLRS). Streptavidin-encoding mRNA does not bind to glutathione Sepharose (SLRS). In addition, when ricin has been inactivated, GST protein-encoding mRNA does not bind to glutathione Sepharose (GLmRS) .
Fig. 12B consists of diagrams showing the amount of GST protein-encoding mRNA recovered post-translation using glutathione Sepharose. After translation, mRNA was bound to glutathione Sepharose, unbound mRNA was washed away, and residual RNA was quantified. After four washes, 1.2% of GLRS mRNA used in the translation had bound to glutathione Sepharose. On the other hand, after four washes, hardly any SLRS and GLmRS mRNA had bound to the agarose (0.2% or less). GLRS mRNA was enriched six times or more after four washes.

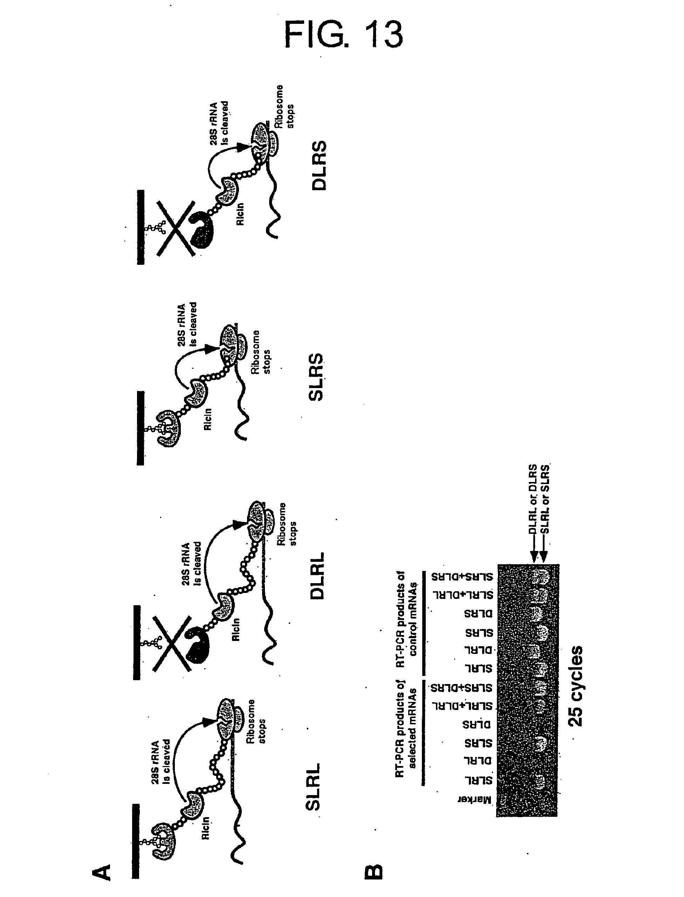

Fig. 13A is a schematic illustration of the selection of streptavidin protein-encoding mRNA using biotin agarose (SLRS and SLRL). DHFR-encoding mRNA does not bind to biotin agarose (DLRS, SLRL). The SLRS and DLRS spacers are shorter than the SLRL and DLRL spacers.
Fig. 13B is a photograph showing the selection of mRNA encoding streptavidin proteins (SLRS and SLRL) from a pool of mixed mRNAs encoding streptavidin and DHFR proteins (SLRS+DLRS and SLRL+DLRL) with biotin agarose. Biotin agarose was added to this mRNA pool after its translation. RT-PCR was then carried out to amplify the bound mRNA. As a result, SLRS mRNA and SLRL mRNA were amplified. On the other hand, SLmRS(L) mRNA and DLRS(L) mRNA were not amplified by RT-PCR. A pool of 1:1 mixture of SLRS(L) mRNA and DLRS(L) mRNA was translated, and biotin agarose was added thereto. SLRS (L) mRNA was revealed to be selectively amplified by RT-PCR. A shorter spacer resulted in more efficient selection.

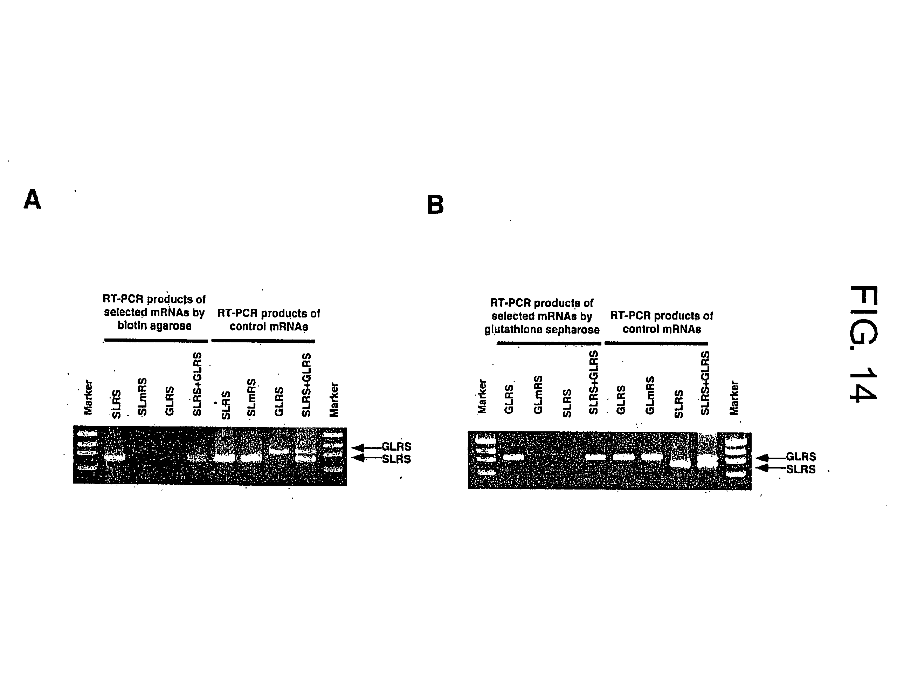

Fig. 14A is a photograph showing the selection of mRNA encoding streptavidin protein (SLRS) from a pool of mixed mRNA encoding streptavidin and GST protein (SLRS+GLRS) with biotin agarose. After translation of SLRS mRNA, biotin agarose was added thereto. RT-PCR was carried out to amplify the bound mRNA. As a result, SLRS mRNA was amplified. On the other hand, SLmRS mRNA and GLRS mRNA were not amplified by RT-PCR. A 1:1 mixed pool of SLRS mRNA and GLRS mRNA was translated, and biotin agarose was added thereto. SLRS mRNA was revealed to be selectively amplified by RT-PCR.
Fig. 14B is a photograph showing the selection of mRNA encoding GST protein (GLRS) from a mixed pool of mRNA encoding streptavidin and GST protein (SLRS+GLRS) with glutathione agarose. After translation of GLRS mRNA, glutathione agarose.was added thereto. RT-PCR was carried out to amplify the bound mRNA. As a result, GLRS mRNA was amplified. On the other hand, GLmRS mRNA and SLRS mRNA were not amplified by RT-PCR. A 1:1 mixed pool of SLRS mRNA and GLRS mRNA was translated, and biotin agarose was added thereto. GLRS mRNA was revealed to be selectively amplified by RT-PCR.

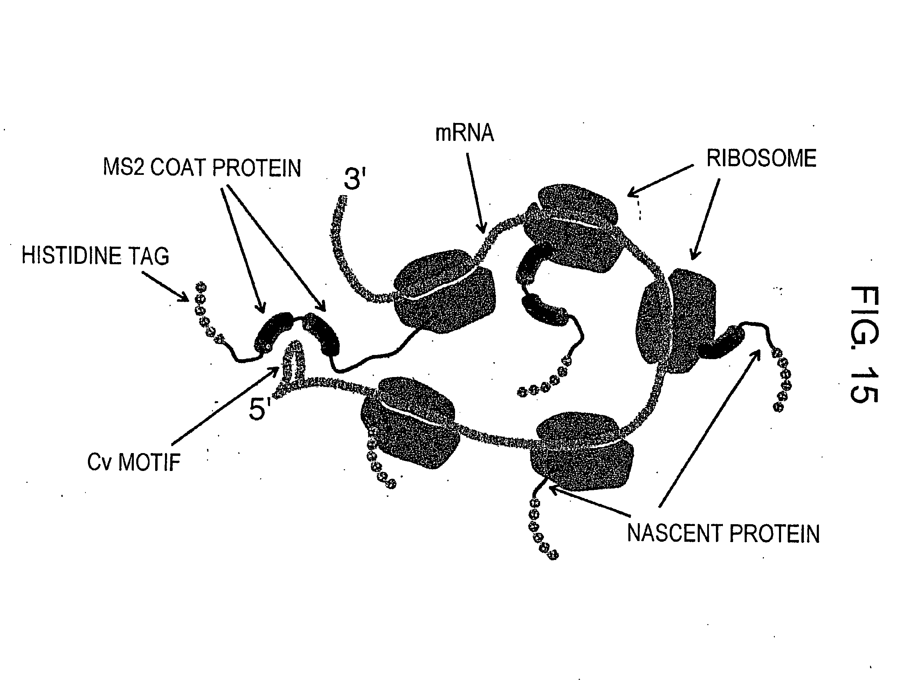

Fig. 15 is a predictive diagram of a circularized polysome display system.

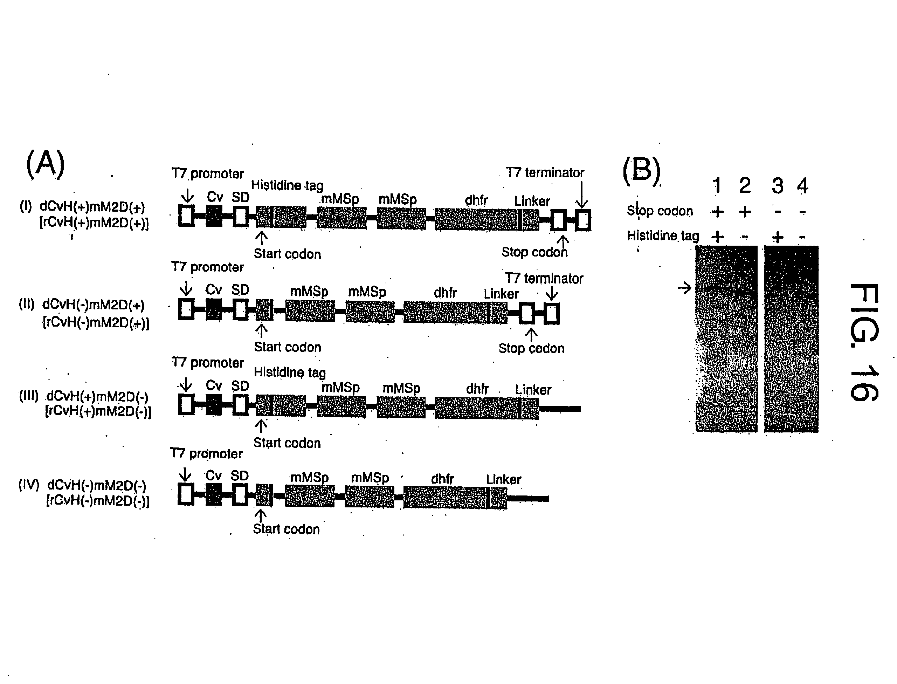

Fig. 16A is a diagram showing the constructs prepared as templates for in-vitro transcription to achieve the novel selection system of the present inventors. The following template DNAs were designed: dCvH (+)mM2D (+) , (I); dCvH(-)mM2D(+),(II); dCvH(+)mM2D(-), (III); and dCvH(-)mM2D (-),(IV). While both dCvH(+)mM2D(+) and dCvH(-)mM2D(+) have the termination codon, dCvH(+)mM2D(-) and dCvH(-)mM2D(-) have no termination codon. The lengths expected were 1786 bp(I), 1723 bp(II), 1716 bp(III) and 1653 bp(IV).
Fig. 16B is a photograph showing the confirmation of *in-vitro* translation. Lane 1, translation product for rCvH(+)mM2D(+); lane 2, translation product for rCvH(-)mM2D(+); lane 3, translation product for rCvH(+)mM2D(-); and lane 4, translation product for rCvH(-)mM2D (-). The predicted molecular weights were 55.7 kDa (lanes 1 and 3) and 53.5 kDa (lanes 2 and 4), respectively.

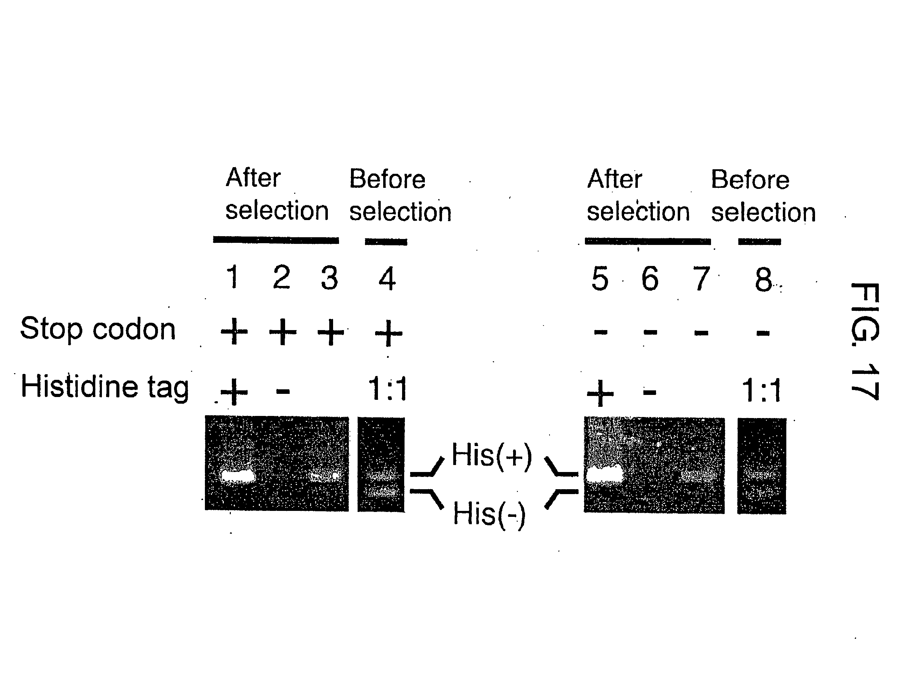

Fig. 17 is a photograph showing the result of *in-vitro* selection to assess the efficiency of the novel system of the present inventors. Patterns of agarose gel electrophoresis for the RT-PCR products are indicated. Lanes 1 to 3 and lanes 5 to 7 contain RT-PCR products amplified from their respective mRNA pools after *in-vitro* selection. For lane 1, the initial mRNA pool contained only rCvH(+)mM2D(+); and for lane 5, the initial mRNA pool contained only rCvH(+)mM2D(-). The lanes indicated above are used as positive controls in the selection. For lane 2, the initial mRNA pool contained only rCvH(-)mM2D(+); and for lane 6, the initial mRNA pool contained only rCvH(-)mM2D(-). These two lanes are used as negative controls in the selection. For lane 3, the initial mRNA pool was a 1:1 mixture of rCvH(+)mM2D(+) and rCvH(-)mM2D(+); and for lane 7, the initial mRNA pool was a 1:1 mixture of rCvH(+)mM2D(-) and rCvH(-)mM2D(-). These two lanes were used for determining selection efficiency. Lane 4 contains RT-PCR products amplified from a 1:1 mixture of rCvH(+)mM2D(+) and rCvH(-)mM2D (+) prior to in-vitro selection; and lane 8 contains RT-PCR products amplified from a 1:1 mixture of rCvH(+)mM2D(-) and rCvH(-)mM2D(-) prior to in-vitro selection.

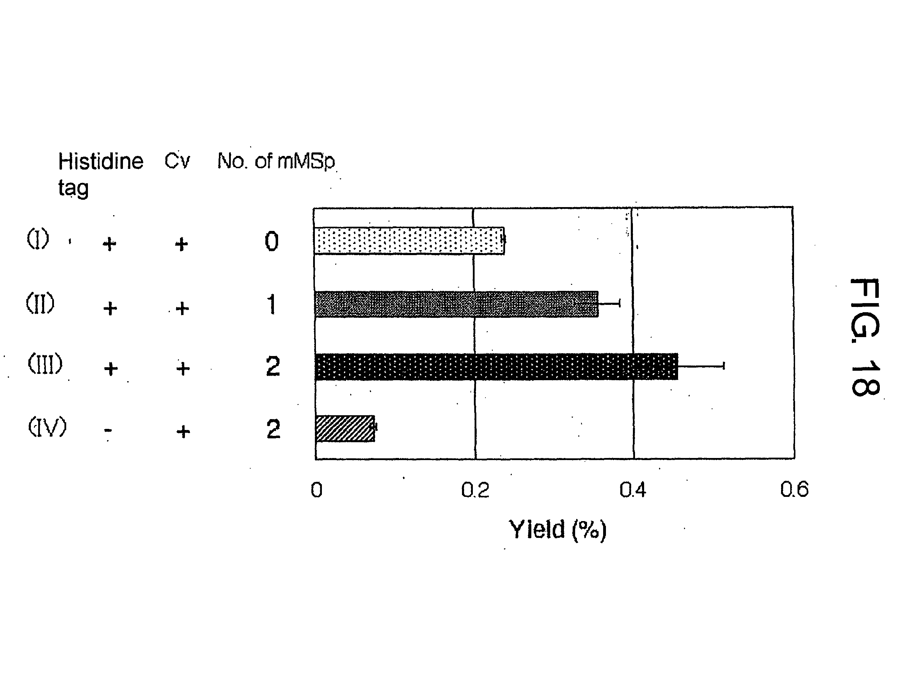

Fig. 18 is a diagram showing the correlation between the involvement of mMS2p-Cv interaction in the system and in-vitro selection. The horizontal axis indicates the yield defined as a ratio between the radioactivity of Ni-NTA agarose pelleted prior to the elution step in the selection, and the radioactivity of the radio-labeled mRNA added. The yield of each mRNA is determined as an average of results obtained in two independent experiments.
   (I) Positive control having the histidine tag. (rCvH(+)D(+)) Contains a Cv but no MSp.
   (II) Positive control having the histidine tag. (rCvH(+)mM1D(+)) Contains a Cv and am MSp.
   (III) Positive control having the histidine tag. (rCvH(+)mM2D(+)) Contains a Cv and two MSps.
   (IV) Negative. control having no histidine tag. (rCvH(-)mM2D(+)) Contains two Cvs and two MSps.

### Best Mode for Carrying out the Invention

The present invention is illustrated in detail below with reference to Examples, but is not to be construed as being limited thereto.

### [Example 1]

### A. Methods

### (A-1) Preparation of three types of proteins [DHFR-(RE)ₙ]

In the preparation of DHFR- (RE)₁₋₃ fusion protein, the DHFR gene derived from plasmid pTZDHFR20 (Blakley, R. L., In Folates and Pterins; Blakley, R. L. ; Benkovic, S. J., Eds.; Wiley: New York, 1985; pp. 191-253) comprising a PstI site at the 3'-end was inserted into plasmid pET30a (Novagen, Madison, WI). Sense and antisense oligonucleotides encoding RE peptide (38 amino acids; RKKRR QRRRP PQGSQ TBQVS LSKQP TSQSR GDPTG PKE (SEQ ID NO: 3)) were synthesized and allowed to anneal. The sequence was elongated using rTaq polymerase (Takara Shuzo Co. , Kyoto, Japan). The resulting dsDNA was double-digested at the *Pst*I and *Eco*T22I sites, and inserted into the PstI site at the 3'-end of the DHFR gene. The restriction sites for *Pst*I and *Eco*T22I were bound to each other. Neither *Pst*I nor *Eco*T22I cleaved the resulting ligated site. Thus, multiple units of RE peptide-encoding sequence were sequentially ligated into the PstI site at the 3'-end of the DHFR gene to give the plasmids: pDHFR-(RE)₁, pDHFR-(RE)₂, and pDHFR-(RE)₃. Cells from *E. coli* NovaBlue (DE3) (Novagen) were transformed with these plasmids, and the resulting transformants were cultured in 500 ml of LB medium at 30°C. After isopropyl-1-thio-β-D-galactoside (IPTG) was added at a concentration of 1 mM during the logarithmic growth phase, the cells were incubated at 30°C for four hours. Proteins were expressed as polypeptides with a poly-histidine tag. The proteins were purified with HisTrap™ chelating kit (Amersham Pharmacia Biotech Uppsala, Sweden) according to the supplier's instructions. Low-molecular-weight materials were removed using a HiTrap™ desalting system (Amersham Pharmacia Biotech). Furthermore, protein degradation products were removed using HiTrap™SP (Amersham Pharmacia Biotech) cation-exchange HPLC. Protein purities were determined using SDS-PAGE. The results are indicated in Fig. 1. The concentrations of the purified proteins were determined using a BCA assay kit (Pierce, Rockford, IL).

### (A-2) Preparation of three types of tandemly arranged RNA aptamers [(Apt)ₙ]

Sense and antisense oligonucleotides (5'-AAA AAA CGA AGC TTG ATC CCG TTT GCC GGT CGA TCG CTT CG-3' (SEQ ID NO: 4) and 5'-TTT TTT CGA AGC GAT CGA CCG GCA AAC GGG ATC AAG CTT C-3' (SEQ ID NO: 5)) were synthesized to prepare the tandemly arranged Tat aptamers [(Apt)₁₋₃]. These oligonucleotides were allowed to anneal, and then ligated using a DNA ligation kit version 2 (Takara Shuzo Co.). Thus, the tandemly arranged Tat aptamers [(Apt)₁₋₃] were yielded. The ligated DNA sequences encoding one to three Tat aptamers were cloned (TA Cloning Kit, Invitrogen Carlsbad, CA) and isolated. The DNA sequences encoding one to three Tat aptamers were transcribed using a T7 Ampliscribe kit (Epicentre Technologies, Madison, WI).

### (A-3) Determination of dissociation constant (K_{d})

Each of the transcribed aptamers [(Apt)₁₋₃], which were to be used in electrophoretic mobility shift assay, was isolated by PAGE using a denaturing gel comprising 15% polyacrylamide and treated with alkaline phosphatase (*E. coli* A19; Takara Syuzo Co., Kyoto, Japan) at 37°C for one hour to dephosphorylate the 5'-ends. Then, the aptamers were labeled with ³²P by incubating with T4 polynucleotide kinase at 37°C for one hour. The DNAs were purified by PAGE using 15% polyacrylamide. 40 nM tRNA was added to a particular concentration of 5'-end-labeled aptamers, and the mixtures were treated in Tat-binding buffer [10 mM Tris-HCl (pH 8.0), 70 mM NaCl, 2 mM EDTA, and 0.1% Nonidet P-40] at 95°C for three minutes for denaturation. Then, the mixtures were allowed to stand at room temperature for renaturation. Excess concentrations of DHFR-(RE)₁₋₃ in the Tat-binding buffer were then added to the mixtures so that the total volumes were 10 µl. The resulting mixtures were incubated at 30°C for one hour or longer. Free RNAs, and RNAs bound to proteins or RNAs bound to peptides were fractionated and analyzed by electrophoresis using a 5 to 15% non-denaturing polyacrylamide gel in 0.5x TBE (Tris-borate/EDTA) buffer.

### (A-4) Construction of pDHFR-(RE)₃-(Apt)₃ (wild type) , pMuDHFR- (RE)₃- Apt)₃ (mutant), and pDHFR-(RE)₃ (control)

The four oligonucleotides (5'-ACA ATT CCC CTC TAG AAA TA-3' (SEQ ID NO: 6), 5'-GTG GTG GTG CTC GAG AAT TC-3' (SEQ ID NO: 7), 5'-CCT GCC GCC CTC GCC TGG GCC AAA CGC AAC ACC TTA AAT AAA-3' (SEQ ID NO: 8), and 5'-GCG TTT GGC CCA GGC GAG GGC GGC AGG CAG GTT CCA CGG-3' (SEQ ID NO: 9)) were prepared to introduce site specific mutations into the core region of DHFR. pMuDHFR-(RE)₃ was constructed by introducing two mutations into the DHFR gene using these oligonucleotides and the Mega Primer PCR mutagenesis. Then, the three tandemly arranged aptamer sequences (Apt)₃ were introduced into pDHFR-(RE)₃ and pMuDHFR-(RE)₃ at the *Eco*RI site downstream of the region for RE peptide. Thus, pDHFR-(RE)₃-(Apt)₃ (wild type) and pMuDHFR-(RE)₃-(Apt)₃ (mutant) were obtained by the procedure described above.

### (A-5) Linkage between a genotype and the phenotype based on the interaction between (Apt)₃ and (RE)₃

The linear plasmids [pDHFR-(RE)₃-(Apt)₃ (wild type), pMuDHFR-(RE)₃-(Apt)₃ (mutant) , and pDHFR-(RE)₃ (control)] were in vitro transcribed using T7 Ampliscribe™ kit (Epicentre Technologies, Madison, WI) at 37°C for two hours to prepare ³²P-labeled capped mRNA (cap analog; New England Bio Labs, Beverly, MA). The labeled mRNAs were purified using NICK™ columns (Amersham Pharmacia Biotech, Uppsala, Sweden). 1 µg of capped mRNA encoding DHFR-(RE)₃ (wild type) or MuDHFR-(RE)₃ (mutant) was translated into 20 µl of rabbit reticulocyte lysate protein (Rabbit Reticulocyte Lysate System; Promega, Madison, WI) at 30°C for 20 minutes. A control mRNA that did not contain aptamer was also translated (control). Each protein was labeled with ³⁵S. After translation, 180 µl of a binding buffer [10 mM sodium phosphate buffer (pH 7.5), 70 mM NaCl, 10 µl tRNA] and 20 µl of a suspension of MTX-agarose beads (Sigma, St Louis, MO) pre-treated with succinic anhydride to neutralize the positive charges were added to the cell lysate. The resulting mixtures were incubated at 4°C for 30 minutes for binding. 200 µl of the supernatant containing unbound mRNA and protein was removed from the mixture, and MTX-beads were washed twice with 180 µl of the buffer. The mRNA and protein bound were eluted from the MTX-beads by incubating in a solution containing SDS at a high temperature (95°C). Then, each sample was fractionated by SDS-PAGE using a 15% gel.

### (A-6) In vitro selection of streptavidin-encoding mRNA from the pool of mixture of streptavidin mRNA and DHFR mRNA

pDHFR- (CQ)₃-(Apt)₃ was constructed by the same procedure as used to construct pDHFR-(RE)₃-(Apt)₃. Briefly, sense and antisense oligonucleotides encoding CQ peptide (36 amino acids: CFTTK ALGIS YGRKK RRQRR RPPQG SQTBQ VSLSK Q) (SEQ ID NO: 10) were synthesized, and then the resulting fragment encoding CQ peptide was inserted between DHFR sequence and aptamer sequence. The template DNA encoding streptavidin has been reported previously. A DNA fragment containing the streptavidin gene was substituted for the DHFR gene upstream of the sequence encoding CQ peptide. Each of the RNAs labeled with ³²P [streptavidin-(CQ)₃-(Apt)₃ and DHFR-(CQ)₃-(Apt)₃] was prepared by the procedure described above. The three combinations of mRNAs (1:1 mixture mRNAs encoding streptavidin and DHFR, and one of the mRNAs to be used as a control) (the total amount = 1.25 µg in each combination) were translated in cell lysate (25 µl) at 30°C for 20 minutes. After translation, 180 µl of the binding buffer [20 mM sodium phosphate buffer solution (pH 8.0), 70 mM NaCl, 10 µl tRNA] and 20 µl of a suspension of biotin-agarose beads (Sigma, St Louis, MO) were added to the cell lysate, and the resulting mixture was incubated on ice for 10 minutes for the binding. 200 µl of the supernatant containing unbound mRNA and protein was removed from the mixture, and then the biotin-beads were washed twice with 180 µl of the buffer. The bond mRNA was eluted from the biotin-beads by incubating in a solution containing urea at a high temperature (95°C). Then, each sample was fractionated by denaturing PAGE using 4% gel.

### (A-7) Combination of ribosome-display system and strong interaction between (RE)₃ and (Apt)₃ in the selection of functional proteins

The above-described plasmids, pET30a and pDHFR-(RE)₃-(Apt)₃ which comprise the DHFR gene (pDHFR) , were used as templates to prepare the ³²P-labled mRNAs [DHFR-(RE)₃-(Apt)₃, DHFR+Stop, and DHFR-Stop mRNA). The ³²P-labeled mRNAs were translated and subjected to the selection with MTX-beads by the same procedure as described above. The bound mRNAs were eluted from the MTX-beads by incubating at a high temperature. The relative amount of the selected RNAs was determined based on the radioactivity of ³²P-labeled mRNA and by RT-PCR.

p(Apt)₃-(RE)₃-DHFR was constructed by the same procedure as used to construct pDHFR- (RE)₃-(Apt)₃. The linear DNA to be used in in-vitro transcription was prepared as a PCR product derived from each template plasmid [p(Apt)₃-(RE)₃-DHFR, or pDHFR] containing T7 promoter sequence. The mRNA was prepared by the same method as described above. Polyclonal IgG antibodies against the respective *E. coli* termination factors (*E. coli* RE1 to RE3) were prepared separately. The total amount of mRNA was 2 µg in the initial pool. Then, the mRNA was in-vitro translated at 37°C for 10 minutes using *E. coli* extract system for linear templates (Promega, Madison, WI). The reaction was allowed to stand on ice to stop the translation. The in-vitro selection using MTX-beads was carried out by the same procedure as described above. The recovered mRNA was purified using RNeasy mini kit (QIAGEN, Hilden, Germany) , and reverse transcription was performed using MMLV reverse transcriptase (Promega). This reverse transcribed sample, namely, cDNA was amplified by PCR (ExTaq; Takara) and then analyzed by electrophoresis using 2.0% agarose. For semi-quantitative analysis, the agarose gel was stained with SYBR Green I (Molecular Probes). The quantitation was carried out using Fluoro Imager 595 (Molecular Dynamics). The intensity of the interaction between (RE)₃ and (Apt)₃ was predicted based on the relative amount of mRNA recovered as RT-PCR products.

### B. Results and Discussion

### (B-1) Potentiation of the interaction between tandemly arranged aptamer and Tat-derived peptide

Dihydrofolate reductase (DHFR) was used as a model for the functional proteins. The reason was that the properties had been well documented (Blakley, R. L. *et al*., New York, 1985, pp.191-253; Taira, K. *et al*., J. Trends Biochem. Sci., 1987, 12, 275-278; Taira, K. *et al*., J. Med. Chem., 1988, 31, 129-137; Iwakura, M. *et al*., J. Biochem. , 1995, 117, 480-488; Fujita, S. *et al*., Proc. Natl. Acad. Sci. USA, 1997, 94, 391-396; and Hamada, M. *et al*., J. Biochem., 1998, 123, 684-692). Three types of proteins [DHFR-(RE)ₙ] (Fig. 1) and three types of tandemly arranged RNA aptamers (Apt)ₙ] (Fig. 2A ) were prepared to assess the influence on the binding affinity between multiple aptamers and Tat-derived peptides (RE). Herein, "n" ranges from one to three, and corresponds to the copy numbers of the respective motifs. Fig. 1 shows the electrophoretic pattern of purified proteins in SDS-PAGE. Each protein was confirmed to have DHFR activity as expected, and it was proven that the binding of RE peptide motif did not inhibit the correct folding of DHFR domain. Tat aptamers were arranged in tandem via six adenosine nucleotides to maintain the proper secondary structure of each aptamer, and thus (Apt)₂ and (Apt)₃ were obtained. An electrophoretic pattern of the purified RNAs in PAGE using non-denaturing gel is shown on lane (C) for a control in Fig. 2A. The pattern in Fig. 2A indicates that Tat aptamer does not interact with DHFR. This electrophoretic pattern shows that the mobility of the aptamer was not altered even when the aptamer was mixed with increased concentrations of wild-type DHFR (in each case, as shown with a narrow triangle above the gel pattern, DHFR concentration was increased from lane C to the right).

The electrophoretic mobility of the aptamers (Apt)₁₋₃ was altered when the aptamers were mixed with fusion proteins DHFR- (RE)₁₋₃ (Figs. 2B to 2D). In this experiment, the ³²P-labeled aptamer (at a final concentration of 30 pM) was mixed with an excess amount of each fusion protein, and the amount of the resulting complex was quantified using an image analyzer. The apparent K_{d} value was determined to be lower than 24 nM, 200 pM, and 16 pM when n=1, n=2, and n=3, respectively. Among the combinations tested, the interaction between (Apt)₃ and DHFR-(RE)₃ was the strongest [K_{d(apt)} < 16 pM]. This complex appeared to have multiple conformations. As both the numbers of aptamers and RE peptides were increased, the interaction became stronger. The maintenance of the stability of RNA-protein complex, namely, very strong interaction between RNA and protein, is an important factor for the linkage between a genotype and the phenotype. Therefore, the experiment described below was carried out using the motifs of (Apt) ₃ and DHFR-(RE)₃.

### (B-2) Linkage between a genotype and the phenotype based on the interaction between (Apt)₃ and (RE)₃

Two types of plasmids were prepared to test.the usefulness of (Apt)₃ and DHFR-(RE)₃. The plasmids encode the wild-type and mutant DHFR-(RE)₃ fusion proteins. The encoding mRNAs and translated proteins are shown in Fig. 3A [wild type, pDHFR-(RE)₃-(Apt)₃; mutant, pMuDHFR-(RE)₃-(Apt)₃].

Each plasmid comprises a sequence encoding DHFR containing tandemly arranged three RE peptides [(RE)₃] and a sequence of tandemly arranged three Tat aptamers (Apt)₃. The mutant protein [MuDHFR-(RE)₃] derived from pMuDHFR-(RE)₃-(Apt-)₃ contains two amino acid substitutions (Ala27 for Asp27 , and Ala31 for Phe31) in the active site of DHFR, and these substitutions discriminate the mutant protein from the wild-type protein [DHFR-(RE)₃] derived from pDHFR-(RE)₃-(Apt)₃. These mutations lack the binding capability to methotrexate (MTX) (Blakley, R. L. *et al*., New York, 1985, pp.191-253; Taira, K. *et al*., J. Trends Biochem., Sci. 1987, 12, 275-278; Taira, K. *et al*., J. Med. Chem., 1988, 31, 129-137; Iwakura, M. *et al*., J. Biochem., 1995, 117, 480-488; Fujita, S. *et al*., Proc. Natl. Acad. Sci. USA, 1997, 94, 391-396; and Hamada, M. *et al*., J. Biochem., 1998, 123, 684-692). The plasmid (pDHFR-(RE)₃) encoding the wild-type DHFR-(RE)₃ fusion protein but encoding no aptamer sequence was prepared as a second control. The translation product DHFR-(RE)₃ cannot interact with the mRNA (Fig. 3A, the right panel).

When the interaction between (Apt)₃ and (RE)₃ can be used for the linkage between a genotype and the phenotype, only in the wild-type system, both protein (DHFR) and mRNA bind to MTX-agarose beads and thus should be recovered after MTX-agarose beads are mixed with the reaction solution after translation (Fig. 3A, the left panel). In contrast, in the mutant system (Fig. 3A, the middle panel) , neither the protein (DHFR) nor the mRNA is recovered when incubated with MTX-agarose beads. Because the mutant DHFR has been designed so as not to bind to MTX. Furthermore, in the second control system (Fig. 3A, the right panel) , only the protein (DHFR) but no mRNA is recovered by incubating with MTX agarose beads. Because DHFR-(RE)₃ cannot interact with the mRNA due to lack of (Apt)₃ aptamer.

Three types of ³²P-labeled capped mRNAs were prepared from the respective plasmids using T7 polymerase. The mRNAs as templates were translated into the respective proteins in rabbit reticulocyte lysate. In the translation, each protein was labeled with ³⁵S-methionine. After translation, an appropriate binding buffer and MTX-agarose beads were added to the respective cell lysates. The resulting mixtures were incubated at 4°C for 30 minutes for the binding (this reaction is carried out at a low temperature to reduce mRNA degradation by RNase in the cell lysate). The supernatant of binding solution·containing unbound mRNA and protein was removed, and then MTX-agarose beads were washed twice with the binding buffer. The products eluted from the beads by heating (95°C) in an elution buffer were analyzed by SDS-PAGE. Each cell lysate after translation was tested, and it was confirmed that the amounts of translated protein and added mRNA agreed in every case (Fig. 3B, lanes 1 to 3).

The products eluted were analyzed. As was expected, DHFR was detectable in the wild-type and control systems (Fig. 3B, lanes 4 and 6) but the mutant protein was almost undetectable (Fig. 3B, lane 5). The recovery rates of wild-type DHFR and control DHFR were nine and 16 times higher than that of mutant DHFR, respectively (Fig. 3C, the left panel). The MTX beads have some positive charges after conjugation with MTX, and it was difficult to completely prevent non-specific binding of mRNA to the MTX beads in this experiment (Fig. 3B, lanes 5 and 6). However, mRNA was detectable only in the wild-type system (Fig. 3B, lane 4). The yield of wild-type mRNA was only 2.5 times higher than the background (specifically, mutant and control mRNAs bound in a non-specific manner) (Fig. 3C, the right panel). The quantitation of the bands revealed that the amounts of protein and mRNA were well above the background level. These results demonstrate that the interaction schematically illustrated in Fig. 3A is effective.

### (B-3) In-vitro selection of mRNA bound to streptavidin

When two discriminable mRNAs are translated simultaneously, each protein binds specifically to the corresponding mRNA, as long as (Apt)₃ and DHFR-(RE)₃ function properly in the selection of target proteins. MTX-agarose beads have positive charges, and thus it is difficult to completely exclude non-specific binding of unrelated mRNAs (Figs. 3B and 3C). For this reason, biotin-agarose beads were used instead. As a positive control plasmid, pStreptavidin-(CQ)₃-(Apt)₃ was newly constructed, which was obtained by substituting of the streptavidin gene for the DHFR gene in pDHFR-(CQ)₃-(Apt)₃ plasmid (both RE and CQ are Tat-derived peptides, and have the same function) (Yamamoto, R. *et al*., Genes Cells, 2000, 5, 371-388). In this system (Fig. 4), a mixture of mRNAs derived from pStreptavidin-(CQ)₃-(Apt)₃ and pDHFR-(CQ)₃-(Apt)₃ is used in the first step, not DHFR but translated streptavidin should bind to biotin-agarose beads (Green, N. M. *et al*., Adv. Protein Chem., 1975, 29, 85-133).

Two types of ³²P-labeled capped mRNAs were prepared from pStreptavidin-(CQ)₃-(Apt)₃ and pDHFR-(CQ)₃-(Apt)₃ using T7 polymerase. The transcribed mRNAs were translated separately in cell lysate (Fig. 4B, lanes 1 and 2), or a 1:1 mixture of the mRNAs was translated (Fig. 4B, lane 3). The respective mRNAs were selected using biotin-agarose beads. This step is essentially the same as the step in the experiment in Fig. 3. After translation, an appropriate binding buffer and biotin-agarose beads were added to each cell lysate, and the mixtures were incubated at 4°C for 10 minutes for the binding. Then, the supernatant of binding solution containing unbound mRNA and protein was discarded, and the biotin-agarose beads were washed twice with the binding buffer. The bound product was eluted from the beads at a high temperature (95°C) using an elution buffer. The selected mRNA was identified by analyzing with 4% denaturing PAGE (Fig. 4B).

Each mRNA bound to the biotin beads was quantitated, and the result showed that the yield of mRNA encoding bound streptavidin (Streptavidin-(CQ)₃-(Apt)₃ mRNA) was three times higher than that of mRNA encoding DHFR (DHFR-(CQ)₃-(Apt)₃ mRNA; lanes 1 and 2). When mRNA bound to biotin beads was isolated from a 1:1 mixture of the mRNAs, pStreptavidin-(CQ)₃-(Apt)₃ mRNA was selected (lane 3). This result demonstrates that the strong RNA-protein interaction is effective to select functional proteins.

### (B-4) Combination of ribosome-display system and the strong interaction between (RE)₃ and (Apt)₃ in the selection of functional proteins

The functional streptavidin was selectable using the strong RNA-protein interaction (Fig. 4). Thus, it was examined whether or not the method can be improved by combining with the ribosome-display system. Since, in the ribosome-display system, the lack of the termination codon delayed the release of protein from the complex (Hanes, J. *et al*., Proc. Natl. Acad. Sci. USA, 1997, 94, 4937-4942.; and He, M. *et al*., Nucleic Acids Res., 1997, 25, 5132-5134) , ribosome forms a relatively stable complex with the translated protein and mRNA. DHFR-mRNA lacking the termination codon (DHFR-Stop mRNA; the right panel in Fig. 5A) was prepared to assess the influence of the lack of the termination codon in the system of the present invention. DHFR mRNA having the termination codon (DHFR+Stop mRNA; the diagram in Fig. 5A) was used as a control.

After translation in cell lysate and subsequent selection, the amounts of mRNA bound to MTX-beads were compared among the three types of mRNAs (specifically, DHFR-(RE)₃-(Apt)₃, DHFR+Stop, and DHFR-Stop). During translation, DHFR-(RE)₃-(Apt)₃ mRNA having the termination codon binds to the translated DHFR protein through the interaction between (RE)₃ and (Apt)₃. Furthermore, ribosome can release from mRNA because of the presence of the termination codon, and thus ribosome does not participate in the formation of the protein-mRNA complex. These three types of mRNAs were translated in rabbit reticulocyte lysate, and the products were exposed to MTX-agarose beads under the same condition as indicated in Fig. 3. The relative amount of mRNA selected was determined based on the radioactivities of the RT-PCR product labeled with ³²P and the original mRNA (Figs. 5B and 5C). The analysis of the PCR products was carried out only once (Fig. 5B), and therefore the result of quantitation of mRNA indicated in Fig. 5C is more reliable. The result shown in Fig. 5C was obtained as an average in independent experiments carried out in quadruplicate. In average, the concentration of DHFR-(RE)₃-(Apt)₃ mRNA selected (Fig. 5C, lane 1) was 2.5 times higher than the background level of DHFR+Stop mRNA (Fig. 5C, lane 2). On the other hand, the concentration of DHFR-Stop mRNA was 1.4 times higher (Fig. 5C, lane 3). Thus, when using this system, the (RE)₃-(Apt)₃ interaction was more effective than the lack of the termination codon in stabilizing the complex between each mRNA and the product thereof.

The ribosome-display method is considerably effective in selecting functional antibodies against antigens (Hanes, J. *et al*., Nat. Biotechnol., 2000, 18, 1287-1292). However, this result (Fig. 5C) suggests that the stability of the complex of ribosome-mRNA-protein formed due to the lack of the termination codon is not sufficient to select an ordinary protein, such as DHFR. A strong antibody-antigen interaction may be required for selection of an appropriate antibody for short time in ribosome-display system.

The combination of (RE)₃-(Apt)₃ interaction and the ribosome-display system is effective in improving the stability of the complex between each mRNA and the product. For this purpose, another plasmid was prepared, in which (Apt)₃-(RE)₃ region is placed upstream of the DHFR gene (Fig. 6). In addition, polyclonal IgG antibodies against the *E*. *coli* termination factors, RF1, RF2, and RF3, were prepared to further stabilize the complex by deleting the termination codon (Moffat, J. G. *et al*., Biochimie, 1991, 73, 1113-1120). The (Apt)₃-(RE)₃ region was placed at the 5'-end so that the termination codon in the (Apt)₃-(RE)₃ region was not located at the 3'-end. Therefore, in the present invention, the SD sequence is placed between (Apt)₃ and (RE)₃, and each mRNA was translated in *E. coil* S30 extract.

The amounts of mRNA bound to MTX-beads in the presence (Fig. 6, lanes 1 and 3) and absence (Fig. 6, lanes 2 and 4) of the polyclonal IgG antibodies against the termination factors were compared between two types of mRNAs; namely, DHFR-Stop mRNA (Fig. 6, lanes 1 and 2) and (Apt)₃-(RE)₃ DHFR-Stop mRNA (Fig. 6, lanes 3 and 4). The translated mRNAs were selected using MTX-agarose beads under the same condition as indicated in Fig. 3. The relative amount of mRNA selected was determined by RT-PCR (Figs. 6B and 6C). The result shown in Fig. 6C was obtained as an average in independent experiments carried out in duplicate. These results demonstrates that the combination of (RE)₃-(Apt)₃ interaction and the ribosome-display system is effective in stabilizing the mRNA-ribosome complex especially in the presence of the polyclonal IgG antibodies against the termination factors.

### (B-5) Possible RNA-protein network

The above-described experimental results demonstrate that the strong interaction of aptamer/peptide. stabilizes the ribosome-display complex and such an interaction is useful in selecting functional proteins. However, it is predictable based on the result of *in-vitro* binding assay (Fig. 2) that the efficiency of the selection of proteins is not very high. Specifically, when the RNA-protein network is formed as illustrated in the upper panel in Fig. 7A, it may be difficult to select proteins from a library. This problem can be overcome using an alternative combination of aptamer and peptide indicated in the bottom panel in Fig. 7A.

### [Example 2]

### A. Materials and Methods

### (A-1) Construction of expression vectors for dihydrofolate reductase (DHFR), glutathione-S-transferase (GST), and streptavidin (StAv) fused with Ricin-A chain

pDHFR was constructed by inserting the DHFR gene derived from PTZDHFR20 into pET30a at the multi-cloning site. Furthermore, two sequences respectively encoding amino acids 1-404 (long spacer) and amino acids 195-315 (short spacer) of the geneIII sequence (for the geneIII sequence, see "*In vitro* selection and evolution of functional proteins by using ribosome display", Proc. Natl. Acad. Sci. USA., 1997 May 13; 94(10):4937-42) derived from pCANTABE5 (Pharmacia) were amplified using the primers described below. The resulting DNAs were digested with *Eco*T22I and *Pst*I, and inserted into the *Pst*I site downstream of the DHFR gene. The primers used are: forward primer for the long spacer: 5'-GTG GCG ATG CAT CGA CTG TTG AAA GTT GTT TAG CAA AAC -3' (SEQ ID NO: 11); reverse primer for the long spacer: 5'-TTC TTA CTG CAG AGA CTC CTT ATT ACG CAG TAT GTT AG-3' (SEQ ID NO: 12); forward primer for the short spacer: 5'-GTG GCG ATG CAT CGG ATC CAT TCG TTT GTG AAT ATC AAG-3' (SEQ ID NO: 13): reverse primer for the short spacer: 5'-TTC TTA CTG CAG ACC AGT AGC ACC ATT ACC ATT AGC AA-3' (SEQ ID NO: 14). Then, the sequence encoding glycine and serine at amino acids 212-258 of the geneIII sequence was amplified as a linker using the primers described below, and digested with *Sca*I and *Eco*RV. The resulting fragment was inserted into the *Eco*RV site between the DHFR gene and the spacer. *Nhe*I and *Nco*I sites were newly inserted upstream and downstream of this linker, respectively. The primers used are: forward primer: 5'-ACT CGG AGT ACT TGC TAG CCC TGT CAA TGC TGG CGG CG-3' (SEQ ID NO: 15); and reverse primer: 5'-GCT ACG GAT ATC CCG CTT GTA ACA GGA GTG CTC CAT GGA TAA TCA AAA TCA CCG GAA CCG -3' (SEQ ID NO: 16). Then, the sequence encoding ricin A chain derived from pUTA (Texas Univ. Prof. Rpbertus J.) was amplified using the primers described below. The products were digested with *Nco*I and *Eco*RV, and inserted between *Nco*I and *Eco*RV between the DHFR gene and the spacer. The primers used are: forward primer: 5'-CGC GCG CCA TGG ATG TTT CCC AAA CAA TAC CCA AT-3' (SEQ ID NO: 17) ; and reverse primer: 5'-GGG GCG GAT ATC CCA AAC TGT GAG CTC GGT GGA GGC GCG-3' (SEQ ID NO: 18). A mutant sequence containing mutations (Glu-177, Arg-180, and Glu-208 were converted to Gln-177, His-180, and Asp-208, respectively), which result in inactivation of ricin, was also inserted (pDLRS and pDLRL) by the same procedure. Finally, to substitute GST and StAv for DHFR, the GST gene derived from pGEX-4t-3 (Pharmacia) and the Streptavidin gene derived from pGSH were amplified, and digested at the *Xba*I and *Nhe*I sites. The resulting fragments were inserted into the plasmid between *Xba*I and *Nhe*I sites (pGLRS, pGLmRS, pSLRS, pSLRL, and pSLmRS). The primers used are: forward primer for GST: 5'-GGC CGG TCT AGA AAT AAT TTT GTT TAA CTT TAA GAA GGA GAT ATA CAT ATG TCC CCT ATA CTA GGT TAT TG (SEQ ID NO: 19); and reverse primer for GST: 5'-CGG TGG GCT AGC CAG ATC CGA TTT TGG AGG ATG GTC GC(SEQ ID NO: 20); forward primer for StAv: 5'-GGC CGG TCT AGA AAT AAT TTT GTT TAA CTT TAA GAA GGA GAT ATA CAT ATG CAC CAT CAT CAT CAT CAT TC -3'(SEQ ID NO: 21); and reverse primer for StAv: 5'-CGG TGG GCT AGC CCG CCG CTC CAG AAT CTC AAA GCA-3'(SEQ ID NO: 22).

The DNA sequences of pSLRL (Streptavidin-Linker-Ricin-GeneIII (long)) and pSLRS (Streptavidin-Linker-Ricin-GeneIII (Short)) are shown in SEQ ID NOs: 1 and 2 in Sequence Listing, respectively.

The nucleotide sequence of SEQ ID NO: 1 comprises the following:
Translation Position, 5076-8155;
T7 promoter, 4987-5004;
lac operator, 5007-5030;
SD sequence, 5061-5067;
Streptavidin sequence, 5121-5600;
Linker, 5601-5768;
Ricin A chain, 5769-6572; and
Spacer (containing GeneIII(L) sequences), 6573-7856.

The nucleotide sequence of SEQ ID NO: 2 comprises the following:
Translation Position, 5076-7007;
T7 promoter, 4987-5004;
lac operator, 5007-5030;
SD sequence, 5061-5067;
Streptavidin sequence, 5121-5600;
Linker, 5601-5768; and
Ricin A chain, 5769-6572.
Spacer (containing GeneIII(S) sequences); 6573-7007

### (A-2) mRNA binding and selection of biotin-affinity column and glutathione-affinity column

Each plasmid (pDLRS, pDLRL, pSLRS, pSLRL, pSLmRS, pGLRS, or pGLmRS) was digested with *Xho*I, and then transcribed with T7 promoter in the presence of a cap analog using AmpliScribe T7 Transcription Kit (Epicentre Technologies). If required, the transcript was radio-labeled with α³²P-CTP. These mRNAs were translated using Rabbit Reticulocyte Lysate System (Promega). The translation was carried out using 1 µg of RNA in a volume of 25 µl. The composition of the solution was the same as described in the attached protocol. The reaction was incubated at 30°C for 10 minutes. 20 µl of the reaction solution was mixed with 80 µl of a sample buffer (50 mM potassium phosphate buffer (pH 7.9), 300 mM NaCl, 0.05% Tween 20, and 2% Block Ace (Yukizirushi) in the case of selection by streptavidin; 10 mM Na₂HPO₄ and 1. 8 mM KH₂PO₄ (pH 7.4), 2.7 mM KCl, 140 mM NaCl, and 1% Triton X-100 in the case of selection by GST selection). 10 µl of Biotin-Agarose (Sigma), which had been pre-washed intensively several times with the sample buffer, was added to the mixture. If required, the amount of RNA bound was determined based on the radioactivity after the steps of binding and washing. After incubation at room temperature for 10 minutes, the supernatant was removed and the beads were washed three times with 200 µl of the sample buffer. Then, to recover the bound RNA, 100 µl of an elution buffer (50 mM Potassium phosphate buffer (pH 7.9), 300 mM NaCl, 0.05% Tween 20, 2% Block Ace, 3M guanidine, and 50 mM EDTA in the selection with streptavidin; and 10 mM Na₂HPO₄ and 1.8 mM KH₂PO₄ (pH 7.4), 2.7 mM KCl, 140 mM NaCl, 1% Triton X-100, 3 M guanidine, and 250 mM EDTA in the selection with GST) was added to the beads. The mixture was stirred vigorously at room temperature for 10 minutes, followed by centrifugation. The resulting supernatant was recovered. This process-was repeated twice. The recovered RNA was purified with RNeasy mini kit (QIAGEN). The purified RNA was reverse transcribed using 5 µM of the reverse primer according to the protocol for Reverse Transcription System (Promega) and amplified by PCR using Ex Taq polymerase (TAKARA). The forward and reverse primers were used at a concentration of 1 µM. The primers used are: forward primer: 5'-CTT TAA GAA GGA GAT ATA CAT ATG-3' (SEQ ID NO: 23) , and reverse primer: 5'-CTC CTC CGG TCG ACG ATA TC-3' (SEQ ID NO: 24).

### B. Results and Discussion

### (B-1) Construction of expression vectors for dihydrofolate reductase (DHFR), glutathione-S-transferase (GST), and streptavidin (StAv) fused with ricin A chain

DHFR, GST, and StAv are proteins whose properties have been well studied. DHFR was inserted to use it as a negative control for the binding between StAv and biotin. The linker was inserted to avoid folding inhibition due to steric hindrance between ricin and DHFR, GST or StAv. This sequence encodes many glycines and serines in GeneIII gene. Not full-length ricin but its A chain, which is the domain having the activity of inactivating ribosome, was inserted. The mutant ricin completely lacked the activity of hydrolyzing the N-glucoside linkage at alpha-salicin site in 28S rRNA. Downstream of ricin, the partial GeneIII sequence was inserted as a spacer. This spacer ensures the ribosome attacking by ricin in a cis-acting manner prior to termination of protein translation by ribosome. Plasmids into which the two types spacers, long and short, had been inserted were constructed respectively (Fig. 9A).

### (B-2) Translation inhibition by ricin A chain

To confirm whether the ricin A chain has the activity of inhibiting the translation by ribosome and. thus can terminate ribosome, the plasmids (pSLmRS, pSLRS, pSLmRL, and pSLRL) constructed as described above were transcribed as template, followed by translation. First, the plasmid was digested at the *Xho*I site downstream of the spacer sequence, the linearized dsDNA was transcribed into mRNA with T7 polymerase. The cap structure was introduced at the 5'-end to ensure the translation in the lysate. Each mRNA of the four types was translated under an.appropriate condition. The resulting proteins were analyzed by SDS-PAGE (Figs. 9B and C). It was revealed that the expression level of fusion protein which comprises a mutant ricin sequence without the activity was higher than that of the fusion protein comprising the wild-type ricin (SLmRS and SLmRL). The amount of protein produced through translation was estimated to decrease because ricin inhibited ribosome. Thus, it was indirectly demonstrated that the ricin fusion protein constructed by the present inventors has the activity of terminating ribosome. In addition, the longer the spacer sequence, the lower the level of protein expression was.

### (B-3) mRNA binding by biotin-affinity column

By the same procedure described above, the plasmids, pDLRS, pSLRS, and pSLmRS, were digested at the *Xho*I site downstream of the spacer sequence, and the linearized dsDNAs were transcribed into mRNAs with T7 polymerase. The cap structure was introduced at the 5'-end to ensure the translation in lysate. The mRNA was labeled with α-CTP to quantify the RNA. Each mRNA of the three types was translated under an appropriate condition, and the resulting products were allowed to bind to biotinylated agarose (biotin-agarose). The biotin agarose is washed several times to remove unbound mRNA. Furthermore, mRNA bound to biotin agarose was quantified in the steps of binding and washing. Biotin has high affinity to StAv.

When DLRS mRNA is translated, ricin terminates ribosome and thus a protein-mRNA complex is formed. However, DHFR does not bind biotin, and therefore it is estimated that almost no mRNA binds to biotin-agarose (the left panel in Fig. 10B). On the other hand, StAv in SLRS mRNA binds to biotin-agarose, and thus mRNA is expected to be recovered (the middle panel in Fig. 10B). Since an inactive ricin has been inserted in SLmRS mRNA, StAv is estimated to binds to biotin-agarose. However, the open reading frame (ORF) contains a termination codon at the end of the mRNA, and therefore it is estimated that the mRNA and protein hardly form a complex (the right panel in Fig. 10B). If only SLRS mRNA is recovered selectively as described above, the system of the present invention is expected to be exceedingly useful in selecting proteins.

mRNA non-specifically bound to the agarose was. removed by repeated washing, and finally SLRS mRNA bound selectively to the agarose (Fig. 10C). 30% of SLRS mRNA bound to the biotin agarose and recovered. This mRNA recovery rate is 2,000 times higher than that (0.015%) by the conventional ribosome-display method, and thus the method of the present invention is expected to be exceedingly useful in securing the variety of random pool. On the other hand, only 0.3% of DLRS mRNA or SLmRS mRNA bound to the agarose. Furthermore, when ricin inactivation decreased, the mRNA recovery rate down to the same level as that of DLRS mRNA. Therefore, ribosome termination by ricin is expected to further stabilize the protein-mRNA complex. Alternatively, RNA may be less degraded in the lysate than in the *E. coli* system. After washing three times, SLRS mRNA is enriched 100 times or more as compared with DLRS mRNA and SLmRS mRNA (Fig. 10C). This efficiency of enrichment is comparable to that achieved by the ribosome-display method. Specifically, it is suggested that the system of the present invention is a system in which a protein can be selected with efficiency comparable to or higher than that of the ribosome-display method.

An alternative selection model was also assessed. GLRS mRNA, SLRS mRNA, and SLmRS mRNA were obtained by transcribing from the plasmids (pGLRS, pSLRS, and pSLmRS) constructed by the procedure described above. The mRNAs were translated according to the same procedure described above. Each of the products was allowed to bind to biotin-agarose (Fig. 11). If the protein selection system works properly, only SLRS mRNA is expected to bind to the biotin-agarose (the middle panel in Fig. 11A). In addition, SLRS mRNA, GLRS mRNA, and GLmRS mRNA were obtained by transcribing from the plasmids (pSLRS, pGLRS, and pGLmRS). The mRNAs were translated according to the same procedure described above. Each of the products was allowed to bind to glutathione-Sepharose (Fig. 12). In this case, only GLRS mRNA is expected to bind to the glutathione-Sepharose (the middle panel in Fig. 12A). mRNA non-specifically bound to the agarose was removed by washing repeatedly, and finally each of SLRS mRNA and GLRS mRNA bound selectively to the Sepharose (Figs. 11B and 12B). In the respective cases, the mRNA recovery rate was 5.1% (biotin-agarose) and 1.3% (glutathione-Sepharose). The efficiency of enrichment was >25 times and >6 times, respectively. Thus, these results suggest that, with the system of the present invention, proteins can be selected with efficiency comparable to or higher than that with the ribosome-display method, and suggest that the method is applicable to various proteins.

### (B-4) Protein selection using biotin-affinity column

As described above, the four types of plasmids (pSLRL, pDLRL, pSLRS, and pDLRS) were transcribed into the four types of mRNAs (SLRL mRNA, DLRL mRNA, SLRS mRNA, and DLRS mRNA). In the cases of SLRL mRNA and DLRL mRNA, the longer spacer was inserted downstream of ricin. Each of the mRNAs was translated under an appropriate condition, and the product was allowed to bind to biotinylated agarose (Fig. 13A). In addition, SLRL mRNA and DLRL mRNA, or SLRS mRNA and DLRS mRNA were mixed at a ratio of 1:1 (0.5 µg each), and translated under an appropriate condition. The products were allowed to bind to biotinylated agarose. The agarose was washed three times, and then the mRNA was separated with the elution buffer. The mRNA was purified and reverse transcribed, followed by amplification by PCR. As a result, a cDNA encoding StAv was selectively amplified by PCR with 15 cycles (Fig. 13B). No cDNA encoding DHFR was amplified by PCR even with 25 cycles. When the 1:1 mixture was used, the StAv-encoding cDNA was amplified selectively. Specifically, it is suggested that StAv protein is properly selected and the gene was recovered with the system of the present invention. It is also suggested that the protein is properly linked with the mRNA of a genotype corresponding to the phenotype. Interestingly, SLRS mRNA bound more efficiently with the agarose as compared with SLRL mRNA.

A fixed amount (0.025 µg) of each mRNA as a control was reverse transcribed, followed by PCR. In addition, SLRL mRNA and DLRL mRNA, or SLRS mRNA and DLRS mRNA were mixed at a ratio of 1:1 (0.0125 µg each), and reverse transcribed, followed by PCR. Thus, it was confirmed that the amplification of cDNA depended on the concentration. In addition, when mRNA was treated only by PCR without the reverse transcription, no cDNA was amplified, and thus no contamination of DNAs was confirmed.

A pool of 1:1 mixture of GLRS mRNA and SLRS mRNA was translated under an appropriate condition according to the same selection method. The products were allowed to bind to biotin-agarose or glutathione-Sepharose. It is estimated that SLRS mRNA was selected when the products bound to biotin-agarose (Fig. 14A) ; and GLRS mRNA was selected when the products bound to glutathione-Sepharose (Fig. 14B). In control experiments, each of SLRS mRNA, SLmRS mRNA, and GLRS mRNA was transcribed, and the products were allowed to bind to biotin-agarose (Fig. 14A), and each of GLRS mRNA, GLmRS mRNA, and SLRS mRNA was transcribed and the products were allowed to bind to glutathione-Sepharose (Fig. 14B). The agarose was washed three times, and then the mRNA was eluted using the elution buffer. This mRNA was purified, and reverse transcribed, followed by amplification by PCR. When the products obtained by translating pool of 1:1 mixture of GLRS mRNA and SLRS mRNA were allowed to bind to biotin-agarose, StAv-encoding cDNA was selectively amplified by PCR. Alternatively, when the same products were allowed to bind to glutathione-Sepharose, GST-encoding cDNA was selectively amplified by PCR. This result also indicates that the protein is properly linked with the mRNA of a genotype corresponding to the phenotype.

### [Example 3]

To assess the general applicability of the ribosome-display system, the present inventors studied the stability of the ribosome-mRNA complex in the presence or absence of the termination codon, and further in the presence or absence of the additional interaction between the translated peptide and the mRNA in the ribosome-mRNA complex.

The additional interaction used by the present inventors was the interaction between a dimer of tandemly fused MS2 coat protein (MSp) and RNA sequence (named C variant (or Cv)) comprising a specific binding motif corresponding to the dimer.

Specifically, the construct shown in Fig. 16 was subjected to *in-vitro* transcription/translation, and the formed complex was selected by Ni-NTA agarose.

Thus, the efficiency of the system was improved by additionally using the mMSp-Cv interaction (Fig. 18). The mRNA recovery rate after selection was 0.46% after washing three times, and was significantly higher than that (0.24%) in the absence of the mMSp-Cv interaction. The present inventors defined the selectivity as the ratio between the amount of histidine tag-encoding mRNA and that of mRNA encoding no histidine tag, which can be assessed by quantitative RT-PCR. The calculated selectivity value was 3 in the absence of Cv, and 6 in the presence of Cv. Thus, the mMSp-Cv interaction improved not only the yield of target mRNA but also the selectivity of the polysome-display system.

### Industrial Applicability

The present invention proved the possibility that the formation of a network structure can be prevented by selecting linkers corresponding to the Tat aptamer and Tat-derived peptides (RE and CQ), and using the very strong interactions between RNA and protein to link a genotype and a phenotype. The use of such an interaction would enable the selection and regulation of a stronger RNA-protein interaction. Furthermore, such an interaction allows the formation of a protein-mRNA complex with a longer half life, thus making selection much easier. In other methods, such as the ribosome-display system, it is difficult to change the original half life of the ribosome-mRNA complex.

Furthermore, the methods of the present invention can be combined with the ribosome-display system by simply removing the termination codon from a fusion gene. As seen in lane 3 in Fig. 6, binding at the sites produces a significantly more stabilized protein-mRNA complex.

In addition, the selection methods of the present invention have the merit of simple and direct manipulation.

When the ricin gene is placed downstream of the StAv gene, a ribosome can be terminated by ricin. The present invention demonstrated that such ribosome termination results in an efficient linkage between genotype and phenotype. Using this method, mRNA of interest can be selected with exceedingly high efficiency. Two types of sequences, long and short, were prepared as the spacer sequence to be placed downstream of the ricin gene. Insertion of the shorter spacer sequence resulted in more efficient mRNA recovery. The recovery rate for SLRS mRNA was 30%, which is a higher recovery rate than ever previously achieved. It was also revealed that this selection system enriched SLRS mRNA 100 times or more, compared with DLRS mRNA.

Novel functional proteins can be selected from a random DNA pool with high efficiency by using the above-described systems of the present invention.

## Claims

1. A DNA construct comprising the DNAs of (i) and (ii), wherein the DNAs are bound so as to express the fused transcript and translation product:
(i) a DNA encoding an arbitrary polypeptide; and
(ii) a DNA encoding a polypeptide having the function of stabilizing a complex comprising the transcript and translation product of the DNA defined in (i).

2. The DNA construct according to claim 1, wherein the DNA defined in (ii) encodes a polypeptide having a ribozyme-inactivating function.

3. The DNA construct according to claim 2, wherein a DNA encoding a spacer peptide is linked downstream of the DNA encoding a polypeptide having the ribozyme-inactivating function.

4. The DNA construct according to claim 2, wherein a DNA encoding a linker peptide is inserted between the DNA encoding the arbitrary polypeptide and the DNA encoding the polypeptide having the ribozyme-inactivating function.

5. The DNA construct according to claim 1, wherein the DNA defined in (ii) is a combination of a DNA encoding an RNA binding protein, and a DNA encoding an RNA to which the RNA binding protein binds.

6. The DNA construct according to claim 5, wherein the fused transcript of the DNAs defined in (i) and (ii) is modified so as to lack the termination codon.

7. The DNA construct according to claim 5, wherein a plurality of DNAs encoding RNA binding proteins are tandemly arranged and wherein a plurality of DNAs encoding RNAs to which the RNA binding proteins bind are tandemly arranged.

8. The nucleic acid construct according to claim 7, wherein the plurality of tandemly arranged DNAs encoding RNA binding proteins are DNAs encoding a plurality of different RNA binding proteins, and wherein the plurality of tandemly arranged DNAs encoding RNAs to which the RNA binding proteins bind, are DNAs encoding RNAs to which a plurality of different RNA binding proteins bind.

9. A nucleic acid construct comprising a complex of an mRNA defined in (i) and a DNA defined in (ii):
(i) a fusion mRNA of an RNA encoding a DNA binding protein, and an RNA encoding an arbitrary polypeptide; and
(ii) a DNA comprising a DNA region to which the translation product of the fusion mRNA defined in (i) binds.

10. The nucleic acid construct according to claim 9, wherein the mRNA defined in (i) is hybridized with the DNA defined in (ii) to form a complex.

11. The nucleic acid construct according to claim 9, wherein the 3'-end region of the mRNA defined in (i) is hybridized with the 3'-end region of the DNA defined in (ii).

12. A DNA construct defined in the following (i) or (ii), which is used for preparing the DNA construct as set forth in claim 1:
(i) a DNA construct comprising a DNA having a function of stabilizing a complex of the transcript and translation product of a DNA encoding an arbitrary polypeptide; and
(ii) a DNA construct comprising a DNA having a function of stabilizing a complex of the transcript and translation product of a DNA encoding an arbitrary polypeptide, and a cloning site for the DNA encoding that arbitrary polypeptide.

13. A method for producing a complex comprising the transcript and translation product of the DNAs defined in (i) and (ii) in the DNA construct as set forth in claim 1, wherein the method comprises expressing the DNAs defined in (i) and (ii).

14. A method for producing a complex of the translation product obtained by translating the mRNA defined in (i) in the nucleic acid construct as set forth in claim 9, and the nucleic acid construct as set forth in claim 9, wherein the method comprises translating the mRNA defined in (i) and binding that translation product to the DNA defined in (ii), in the nucleic acid construct as set forth in claim 9.

15. The method according to claim 14, wherein the method uses a nucleic acid construct in which a complex is formed by hybridizing the mRNA defined in (i) and the DNA defined in (ii).

16. The method according to claim 14, wherein the method uses a nucleic acid construct in which the 3'-end region of the mRNA defined in (i) is hybridized with the 3'-end region of the DNA defined in (ii).

17. A method for elongating the 3'-end region of the DNA defined in (ii) in a complex produced by the method as set forth in claim 15, wherein the method comprises contacting the complex with reverse transcriptase and carrying out a reverse-transcription reaction using the mRNA defined in (i) as a template.

18. A method for elongating the DNA defined in (ii) in a complex produced by the method as set forth in claim 16, wherein the method comprises contacting the complex with reverse'transcriptase and carrying out reverse-transcription reaction using the mRNA defined in (i) as a template and the DNA defined in (ii) as a primer.

19. The method according to any one of claims 13 to 18, wherein the method is used in a cell-free system.

20. A complex produced by the method as set forth in claim 13.

21. A complex produced by the method as set forth in any one of claims 14 to 18.

22. A method of screening for a polypeptide binding to a particular target substance, or for an mRNA encoding that polypeptide, wherein the method comprises the steps of:
(a) forming a complex comprising the transcript and translation product of the DNAs defined in (i) and (ii) in the DNA construct as set forth in claim 1, by expressing the DNAs defined in (i) and (ii);
(b) contacting the particular target substance with the complex formed in step (a); and
(c) collecting the complex bound to that target substance.

23. A method of screening for a polypeptide binding to a particular target substance, or for an mRNA encoding that polypeptide, wherein the method comprises the steps of:
(a) translating the mRNA defined in (i) in the nucleic acid construct as set forth in claim 9,
(a') forming a complex comprising this translation product and the nucleic acid construct as set forth in claim 9, by binding the translation product to the DNA defined in (ii), in the nucleic acid construct as set forth in claim 9;
(b) contacting the particular target substance with the complex formed in step (a); and
(c) collecting the complex bound to that target substance.

24. A method of screening for a polypeptide binding to a particular target substance or for an mRNA or a DNA encoding the polypeptide, wherein the method comprises the steps of:
(a) translating the mRNA defined in (i) in the nucleic acid construct as set forth in claim 10,
(a') forming a complex comprising that translation product and the nucleic acid construct as set forth in claim 10, by binding the translation product to the DNA defined in (ii), in the nucleic acid construct as set forth in claim 10;
(b) elongating the DNA defined in (ii) in the complex formed in step (a), by contacting the complex with reverse transcriptase and carrying out a reverse-transcription reaction using, as a template, the mRNA defined in (i) in the complex;
(c) contacting the particular target substance with the complex formed in step (b); and
(d) collecting the complex bound to that target substance.

25. A method of screening for a polypeptide binding to a particular target substance or for an mRNA or DNA encoding that polypeptide, wherein the method comprises the steps of:
(a) translating the mRNA defined in (i) in the nucleic acid construct as set forth in claim 11,
(a') forming a complex comprising the translation product and the nucleic acid construct as set forth in claim 11, by binding the translation product to the DNA defined in (ii) in the nucleic acid construct as set forth in claim 11;
(b) elongating the 3'-end region of the DNA defined in (ii) in the complex formed in step (a), by contacting the complex with reverse transcriptase and carrying out a reverse-transcription reaction using as a template the mRNA defined in (i) in the complex, and as a primer the DNA defined in (ii) in the complex;
(c) contacting the particular target substance with the complex formed in step (b); and
(d) collecting the complex bound to that target substance.

26. A method of screening for a polypeptide binding to a particular target substance or for an mRNA or DNA encoding that polypeptide, wherein the method comprises the steps of:
(a) translating the mRNA defined in (i) in the nucleic acid construct as set forth in claim 10,
(a') forming a complex comprising the translation product and the nucleic acid construct as set forth in claim 10, by binding the translation product to the DNA defined in (ii) in the nucleic acid construct as set forth in claim 10;
(b) contacting the particular target substance with the complex formed in step (a);
(c) elongating the DNA defined in (ii) in the complex bound to the target substance, by contacting the complex with reverse transcriptase and carrying out reverse-transcription reaction using, as template, the mRNA defined in (i) in the complex; and
(d) collecting the complex bound to the target substance.

27. A method of screening for a polypeptide binding to a particular target substance, or for an mRNA or a DNA encoding that polypeptide, wherein the method comprises the steps of:
(a) translating the mRNA defined in (i). in the nucleic acid construct as set forth in claim 11,
(a') forming a complex comprising the translation product and the nucleic acid construct as set forth in claim 11, by binding the translation product to the DNA defined in (ii) in the nucleic acid construct as set forth in claim 11;
(b) contacting the particular target substance with the complex-formed in step (a);
(c) elongating the 3'-end region of the DNA defined in (ii) in the complex bound to the target substance, by contacting the complex with reverse transcriptase and carrying out a reverse-transcription reaction using, as a template, the mRNA defined in (i) in the complex, and, as a primer, the DNA defined in (ii) in the complex; and
(d) collecting the complex bound to the target substance.

28. The method according to any one of claims 22 to 27, wherein the target substance is fixed on a carrier.

29. A kit used for the screening method as set forth in claim 21, wherein the kit comprises the DNA construct as set forth in claim 1 or 12, or the complex as set forth in claim 20.

30. A kit used for the screening method as set forth in any one of claims 23 to 27, wherein the kit comprises the nucleic acid construct as set forth in claim 9, or the complex as set forth in claim 21.
